# EUROPEAN PATENT APPLICATION

(11) **EP 4 814 022 A1**
(43) Date of publication of application: **30.09.2026**
(21) Application number: 25166053.6
(22) Date of filing: 25.03.2025
(51) Int. Cl.: C08F 8/50, C08J 11/04, C08L 55/02, C08L 69/00

(54) **COMPOSITION COMPRISING A RUBBER-MODIFIED GRAFT POLYMER, METHANOL, AN AROMATIC DIHYDROXY COMPOUND AND SODIUM**

(71) Applicant: Covestro Deutschland AG, 51373 Leverkusen (DE)
(72) Inventor: YOGENDRA, Sivathmeehan, 40211 Düsseldorf (DE); SLUYTS, Erik, 2930 Brasschaat (DE); SEIDEL, Andreas, 41542 Dormagen (DE)
(74) Representative: Levpat

(57) **Abstract**

The present invention relates to a composition comprising a rubber-modified graft polymer and optionally a rubber-free vinyl(co)polymer, comprising more than 0 ppm methanol, more than 0 ppm of at least one aromatic dihydroxy compound and less than 1000 ppm sodium. Furthermore, the present invention relates to a composition comprising the inventive composition and at least one blend partner and/or a polymer additive. Moreover, the present invention relates to a thermoplastic molding composition and a molded article.

## Description

The present invention relates to a composition comprising a rubber-modified graft polymer and optionally a rubber-free vinyl(co)polymer, comprising more than 0 ppm methanol, more than 0 ppm of at least one aromatic dihydroxy compound and less than 1000 ppm sodium. Furthermore, the present invention relates to a composition comprising the inventive composition and at least one blend partner and/or a polymer additive. Moreover, the present invention relates to a thermoplastic molding composition and a molded article.

Polycarbonates (PC) are applied diversly in industry and everyday life. They are known for their good property profile in terms of mechanical and optical properties, temperature resistance, and weathering stability. Due to this property profile, polycarbonates have long been used in a wide variety of indoor and outdoor applications, for example for the automotive sector, for rail vehicles, for the construction sector, for the electrical/electronics/IT sector, for battery housings for household appliances, for the lighting sector or for the medical technology sector. The property profile of polycarbonate may further be adjusted to the demands of the respective use via the selection of additional polymeric components added to the polycarbonate and the ranges of amounts in which these components are used in the compositions.

To increase notch resistance, in particular at low temperatures, blending partners having elastomeric properties are added to the polycarbonate as impact modifiers. The impact modifiers in turn may differ in the chemical composition of the elastic component or else the morphology thereof. An important class of impact modifiers are rubber-modified graft polymers. They can be blended with polycarbonate in a compounding process and a two-phase morphology is created. In most cases the phase morphology of these polycarbonate blends is such that polycarbonate forms the matrix phase and the rubber-modified graft polymer forms domains within the matrix phase.

Typically, such rubber-modified graft polymers can additionally comprise at least one vinyl(co)polymer which is not bound to the rubber. Amongst those, styrene-acrylonitrile copolymers (SAN) combined with polybutadiene-based rubber particles, commonly known as acrylonitrile-butadiene-styrene (ABS), is the most prominent component. It is used on a large scale as blending partner with incorporated impact modifier for polycarbonate compositions. In this combination the rubber particles improve notched impact resistance and further mechanical properties, while SAN facilitates the melt flowability of the resulting blend with polycarbonate and hence facilitates processing to give molded articles.

As a result of the range of application possibilities due to a versatile and adjustable property profile, and the associated economic success, large quantities of waste comprising at least polycarbonate are generated. Such polycarbonate waste which may comprise additional blend partners must be put to meaningful use. The technically simplest type of use is incineration, utilizing the released combustion heat for other processes, such as industrial manufacturing processes. However, this method does not allow for the closure of raw material cycles. Another type of use is typically subsumed under the expression "physical recycling," in which polycarbonate waste is mechanically shredded and used in the production of new products. This type of recycling naturally has its limits, which is why there have been numerous attempts to recover the raw materials underlying polycarbonate production by cleaving the polycarbonate bonds (typically referred to as "chemical recycling"). The raw materials to be recovered usually include bisphenols, especially bisphenol A. Depending on the type of chemical recycling, a carbonate-containing compound such as diphenyl carbonate or dimethyl carbonate, or even CO₂, can also be obtained. In case of compositions comprising polycarbonate and at least one polymeric blend partner such as for example ABS or PMMA this polymeric blend partner would be also a valuable raw material and thus, it would be desirable to recycle it.

WO 2014/099594 A1 relates to a method for recovering a dihydroxy aromatic compound and a dialkyl carbonate from a composition comprising a polycarbonate and acrylonitrile-butadiene-styrene. The method comprises depolymerizing the polycarbonate producing a dihydroxy aromatic compound and a dialkyl carbonate; removing the dihydroxy aromatic compound and the dialkyl carbonate from the reactor, the acrylonitrile-butadiene-styrene remaining as a coating on the surfaces of the reactor; adding a solvent like acetone to the reactor; and heating the solvent to remove the acrylonitrile-butadiene-styrene from the surfaces of the reactor. In the examples of this document no sodium catalyst is used. Accordingly, the resulting acrylonitrile-butadiene-styrene is free of sodium.

WO 2023/110654 A1 describes a process for the recovery of a rubber-modified vinyl(co)polymer from a polycarbonate resin comprising polycarbonate and a rubber-modified vinyl(co)polymer. The method comprises the step of bringing the polycarbonate resin into contact with an alcohol, water and a transesterification catalyst to obtain a slurry. By keeping the temperature below the softening temperature of the rubber-modified vinyl(co)polymer, i.e. at 60 to 90 °C, the rubber-modified vinyl(co)polymer is present as hard particles, which may be separated by filtration from the liquid fraction of the slurry comprising the diol resulting from the depolymerization of the polycarbonate.

The mentioned documents relate to the depolymerization of polycarbonate compositions or compounds to obtain the polycarbonate building blocks which are then suited for producing new polycarbonates. In case an additional polymeric component is present this component is either separated prior to polycarbonate depolymerization, is present as solid in a slurry, remains as coating on the reactor equipment which needs to be removed in a last step.

Only few of the chemical recycling processes known in the literature are currently operated on an industrial scale. This is particularly true for the chemical recycling of compositions and articles (especially end of life articles) not solely comprising polycarbonate but also additional polymeric components as blend partners, additives or coatings. This is mainly due to the lack of process efficiency in terms of limited product yield, the high complexity of processes and/or the limited quality of the reaction products obtained.

It is for example known from the prior art that the methanolysis of PC/ABS blends forms sticky ABS residue in the product mixture. The sticky material complicates subsequent handling and processing, leading to reduced yields of recyclable materials and increased process complexity. This reduces the overall efficiency of the recycling process.

Based on this prior art, it was an object of the present invention to improve at least one known disadvantage. Especially, there was need to develop an efficient and scalable process for the recycling of rubber-modified graft polymers optionally in mixture with a rubber-free vinyl(co)polymer which yields the rubber-modified graft polymer optionally in mixture with a rubber-free vinyl(co)polymer in high quality. This is preferably understood that the recycled rubber-modified graft polymer optionally in mixture with a rubber-free vinyl(co)polymer can be used in compositions, e. g. in compounding with other polymeric component(s) and/or polymer additives. More preferably, this implies that it has minimal negative impact on other polymeric component(s) and/or additive. For example, specific impurities such as sodium are known to degrade other polymeric component(s) such as polycarbonate and lead to the reduction of mechanical properties as well as cause color in the resulting blend. At the same time it would be desirable to obtain a recovered rubber-modified graft polymer optionally in mixture with a rubber-free vinyl(co)polymer which maintains its basic properties such as low glass transition temperature of the rubber phase. By this the recovered material keeps it elastic properties and is usable for standard applications of this material class.

At least one of the above-mentioned objects, preferably all of these objects have been solved by the present invention.

Surprisingly, it was found that the use of the recycling process of a methanolysis with defined amounts of catalyst provides for effective means to solve at least one, preferably all of the above-mentioned objects. According to the present invention, a process is used in which the ratios of the components in the reaction mixture of the methanolysis of an article comprising polycarbonate comprising a structural unit derived from an aromatic dihydroxy compound and at least one vinyl(co)polymer and/or a rubber-modified graft polymer are chosen in a way so that the vinyl(co)polymer and/or rubber-modified graft polymer is completely in the liquid phase (at least at the end of the methanolysis reaction of inventive step (ii)). The process parameters prevent the formation of a sticky vinyl(co)polymer/rubber-modified graft polymer residue and at the same time allow the efficient depolymerization of PC, recovery of its monomers and recovery of the rubber-modified graft polymer optionally in mixture with a rubber-free vinyl(co)polymer. It was found that the aromatic dihydroxy compound sticks to the at least one vinyl(co)polymer and/or a rubber-modified graft polymer. However, the amount of aromatic dihydroxy compound which can be removed from the rubber-modified graft polymer was found to be different for a process in which the rubber-modified graft polymer is in the liquid phase when compared to a process where it is still solid / forms a sticky mass. When washing this aromatic dihydroxy compound cannot be easily removed from the rubber-modified graft polymer which is solid / forms a sticky mass during methanolysis. This means that a high number of washing steps would be required in order to obtain a rubber-modified graft polymer with high purity which reduces the efficiency of the whole process. However, when using the inventive process the aromatic dihydroxy compound can be recovered (e.g. washed) from the rubber-modified graft polymer more easily. This means that less washing steps are needed to get an economically reasonable process. Moreover, this means that a high purity of the rubber-modified graft polymer can be obtained using less solvent for washing and / or less purification steps for recovering resulting in less energy consumption. Moreover, the resulting rubber-modified graft polymer comprises less aromatic dihydroxy compounds especially when referring to the same effort of washing. Moreover, it was found that when using specific amounts of catalyst, e.g. especially sodium containing catalyst, the resulting rubber-modified graft polymer comprises a reasonable amount of sodium while at the same time the methanolysis reaction efficiently degrades the polycarbonate. In this context the term "reasonable amount of sodium" means that the amount of sodium is acceptable, because it minimizes the negative impact on other polymeric component(s) and/or additive when the rubber-modified graft polymer containing such reasonable amounts of sodium is compounded with other polymeric partner(s) and/or additives. Especially, it was found that the basic properties such as low glass transition temperature of the rubber phase of the rubber-modified graft polymer is maintained when using the specific inventive process. By this the recovered material keeps it elastic properties and is usable for standard applications of this material class.

Finally, the inventive process was found to be scalable and at the same time ecologically and/or economically effective. It was found that the rubber-modified graft polymer can be precipitated in a way that it is filterable and can be easily removed from the (other) reaction products. This filtration needs low pressures and is very quick. As said above, it results in a rubber-modified graft polymer which has high purity, especially a reasonable amount of sodium. This is especially true when compared to the number of washing steps.

According to the present invention, when referring to "high purity" of a rubber-modified graft polymer this preferably means that it has low amounts of monomeric components such as aromatic dihydroxy compounds and/or vinyl monomers, low amounts of sodium, low amounts of methanol and/or low amounts of dimethyl carbonate. More preferably "high purity" of a rubber-modified graft polymer this preferably means that it has low amounts of monomeric components such as aromatic dihydroxy compounds, , 1,3-butadiene, acrylonitrile, 4-vinyl-1-cyclohexane or styrol, low amounts of sodium, low amounts of methanol, low amounts of dimethyl carbonate, low amounts of ethylbenzene and / or low amounts of chlorobenzene.

Accordingly, the present invention provides a composition comprising a rubber-modified graft polymer B.1 and optionally a rubber-free vinyl(co)polymer B.2, comprising more than 0 ppm methanol, more than 0 ppm of at least one aromatic dihydroxy compound and more than 0 ppm and less than 1000 ppm sodium, wherein the ppm are based on the amount of the sum of B.1 and B.2.

It is preferred that the amount of methanol in the inventive composition is more than 0.1 ppm and equal to or less than 10 000 ppm. More preferably, the amount of methanol in the inventive composition is 0.5 ppm to 9 000 ppm, still preferably 1 ppm to 7 500 ppm, still preferably 2 ppm to 5 000 ppm, still preferably 5 ppm to 2 500 ppm, still preferably 8 ppm to 1 000 ppm, still preferably 10 ppm to 750 ppm, still preferably 15 ppm to 500 ppm and most preferably 20 ppm to 300 ppm.

The skilled person is capable of determining the amount of MeOH in the inventive composition with method known in the art. Preferably, the amount of MeOH in the inventive composition is determined using gas chromatography.

Furthermore, it is preferred that the amount of the at least one aromatic dihydroxy compound in the inventive composition is more than 1 ppm and equal to or less than 10 000 ppm. More preferably, the amount of the at least one aromatic dihydroxy compound in the inventive composition is 1.5 ppm to 9 000 ppm, still preferably 2 ppm to 8 000 ppm, still preferably 3 ppm to 7 500 ppm, still preferably 5 ppm to 7 000 ppm, still preferably 8 ppm to 6 000 ppm, still preferably 10 ppm to 5 000 ppm, still preferably 20 ppm to 4 000 ppm, still preferably 50 ppm to 3 500 ppm, still preferably 100 ppm to 3 000 ppm, still preferably 150 ppm to 2 500 ppm and most preferably 200 ppm to 2 000 ppm. Due to regulations it might be also beneficial if the inventive composition comprises 0.1 to less than 10 ppm and most preferably 1 ppm to less than 150 ppm of the at least one aromatic dihydroxy compound.

Preferably, the at least one aromatic dihydroxy compound comprises, more preferably is a compound selected from the group consisting of formula (1) and (IV) to (IX), wherein 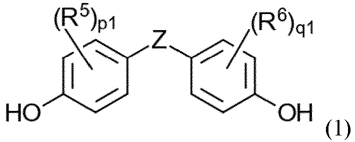 wherein each Z independently represents a single bond, -S(=O)₂-, -C(=O)-, -O-, -S-, -S(=O)-, -CH(CN)-, linear or branched C₁-C₆-alkylene which optionally comprises at least one carbonyl-group, optionally comprises at least one halogen atom and/or optionally is interrupted by at least one heteroatom, C₂-C₁₀-alkylidene which optionally comprises at least one carbon-carbon-double bond, optionally comprises at least one carbonyl-group and/or optionally comprises at least one halogen atom, C₅-C₁₂-cycloalkylene, wherein the cycloaliphatic group is fused to at least one further cycloaliphatic ring, C₅-C₁₅-cycloalkylidene wherein the cycloaliphatic group is optionally fused to at least one cycloaliphatic and /or at least one aromatic ring, C₇-C₂₀-aralkylidene, C₈-C₂₀-aralkylene, C₆-C₁₂-arylene optionally being fused to further aromatic rings which optionally may comprise at least one hetero atom, formula (B1-A), formula (B1-B), formula (B1-C) or formula (B1-D) 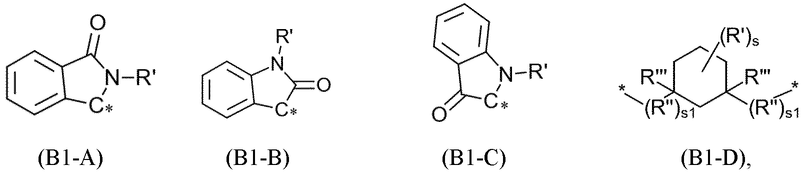
wherein in formulae (B1-A), (B1-B) and (B1-C) each R' independently represents a linear C₁-C₄-alkyl, branched C₃-C₄-alkyl, aralkyl or aryl and the "C*" indicates the quaternary carbon atom which is at the position indicated as "Z" in formula (1), and
wherein in formula (B1-D) each R' independently represents a linear C₁-C₄-alkyl, branched C₃-C₄-alkyl, each R" independently represents a linear C₁-C₄-alkylene, branched C₃-C₄-alkylene, each R‴ independently represents H or linear C₁-C₃-alkyl, s is 0 to 2 and each s1 independently is 0 or 1, and the "*" indicate the bonds which link "Z" to the aromatic rings in formula (1),
each R⁵ and R⁶ independently represents H, C₁-C₁₈-alkyl, C₅-C₆-cycloalkyl, C₁-C₅-alkenyl-, C₁-C₁₈-alkoxy, phenoxy, halogen atom, C₆-C₁₈-aryl or C₇-C₁₈-aralkyl, and
each of p1 and q1 is 1 to 4, 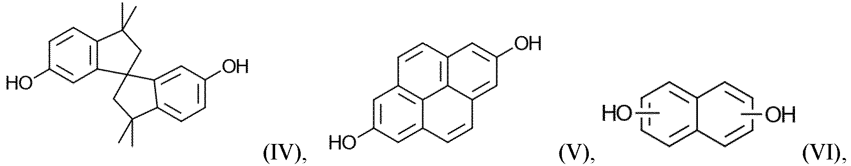 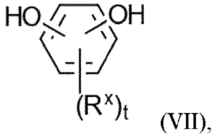 wherein in formula (VII) each R^{x} independently represents linear or branched C₁-C₆-alkyl, C₁-C₁₂-aryl, C₁-C₈-aralkyl or a halogen atom and t is 0 to 4, 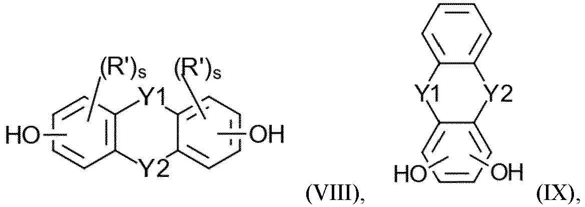 wherein in formulae (VIII) and (IX) each Y1 independently represents oxygen, sulfur or N-R^{y}, wherein R^{y} is H or -CH₃, each Y2 interpedently represents a single bond, oxygen, sulfur or N-R^{y} as defined above and in formula (VIII) each R' independently represents a linear C₁-C₄-alkyl, branched C₃-C₄ alkyl, aralkyl or aryl and s is 0 to 2.

More preferably, the at least one aromatic dihydroxy compound is a bisphenol. Most preferably, the aromatic dihydroxy compound is a compound of formula (1).

Still preferably, the aromatic dihydroxy compound is a compound of formula (1), wherein each Z independently represents a single bond, -S(=O)₂-, -C(=O)-, -O-, -S-, -S(=O)-, linear or branched C₁-C₆-alkylene, C₂-C₁₀-alkylidene which optionally comprises at least one carbon-carbon-double bond, C₅-C₁₅-cycloalkylidene wherein the cycloaliphatic group is optionally fused to at least one cycloaliphatic and /or at least one aromatic ring, C₇-C₁₅-aralkylidene and the C₈-C₁₅-aralkylene of formula (B1-E) 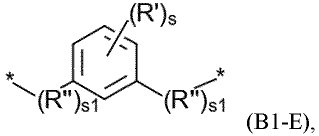
wherein in formula (B1-E) each R' independently represents a linear C₁-C₄-alkyl, branched C₃-C₄ alkyl, each R" independently represents a linear C₁-C₄-alkylene, branched C₃-C₄ alkyl, s is 0 to 2 and each s1 independently is 0 or 1, and the "*" indicate the bonds which link "Z" to the aromatic rings in formula (1),
formula (B1-A), formula (B1-B), formula (B1-C),
each R⁵ and R⁶ independently represents H, C₁-C₁₈-alkyl, C₅-C₆-cycloalkyl, C₁-C₁₈-alkoxy, phenoxy, C₆-C₁₈-aryl or C₇-C₁₈-aralkyl and
each of p1 and q1 is 1 to 4.

More preferably, the aromatic dihydroxy compound comprises at least one compound of formula (1), wherein each Z independently represents a single bond, C₂-C₆-alkylidene, C₅-C₁₂-cycloalkylidene wherein the cycloaliphatic group is optionally fused to at least one aromatic ring,
formula (B1-A), formula (B1-B), formula (B1-C),
each R⁵ and R⁶ independently represents H, C₁-C₃-alkyl, C₁-C₁₈-alkoxy,
each of p1 and q1 is 1 to 4.

Still more preferably, the aromatic dihydroxy compound is a compound of formulae (1a) or (1b)

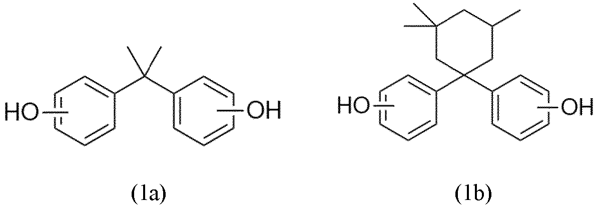

Most preferably, the aromatic dihydroxy compound is a compound of formula (1a). According to the present invention, this compound is also referred to as bisphenol A or BPA. In some cases in the context of the present invention reference is made to a "dihydroxy compound". This intrinsically means that this dihydroxy compound is aromatic.

Most preferably, the aromatic dihydroxy compound is a mixture of at least 20 wt.-%, more preferably at least 25 wt.-% and most preferably at least 50 wt.-% of a compound of formula (1a) and another compound of formula (1) being distinct from formula (1a), wherein the wt.-% refer to the weight of all aromatic dihydroxy compounds which are present in the inventive composition.

It is particularly preferred that the amount of bisphenol A in the inventive composition is more than 1 ppm and equal to or less than 10 000 ppm. More preferably, the amount of bisphenol A in the inventive composition is 1.5 ppm to 9 000 ppm, still preferably 2 ppm to 8 000 ppm, still preferably 3 ppm to 7 500 ppm, still preferably 5 ppm to 7 000 ppm, still preferably 8 ppm to 6 000 ppm, still preferably 10 ppm to 5 000 ppm, still preferably 20 ppm to 4 000 ppm, still preferably 50 ppm to 3 500 ppm, still preferably 100 ppm to 3 000 ppm, still preferably 150 ppm to 2 500 ppm and most preferably 200 ppm to 2 000 ppm. Due to regulations it might be also beneficial if the inventive composition comprises 0.1 to less than 10 ppm and most preferably 1 ppm to less than 150 ppm of bisphenol A.

Preferably, the amount of the aromatic dihydroxy compound, preferably bisphenol A in the inventive composition is determined using gas chromatography or HPLC. Most preferably, the amount of the aromatic dihydroxy compound, preferably bisphenol A in the inventive composition is determined using gas chromatography.

"Alkyl" in the context of the present invention, for example and if not mentioned differently, refers to an alkane structure of which one hydrogen atom is removed. The "alkyl" of the present invention which can be linear or branched is saturated and therefore, it comprises only single bonds between adjacent carbon atoms. Preferably, alkyl groups according to the present invention comprise methyl, ethyl, *n*-propyl, isopropyl, *n*-butyl, *sec*-butyl, *tert*-butyl, *n*-pentyl, 1-methylbutyl, 2-methylbutyl, 3-methylbutyl, neopentyl, 1-ethylpropyl, *n*-hexyl, 1,1-dimethylpropyl, 1,2-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, 1-ethyl-2-methylpropyl and the like. These structures can be limited in choice, in case the invention defines the carbon atoms of an alkyl group in a different manner.

"Alkylene" in the context of the present invention, for example and if not mentioned differently, refers to a bridging alkane structure of which two hydrogen atoms from different carbon atoms are removed. In this context the two carbon atoms of which the two hydrogens atoms are removed can be removed from any carbon atom which is present in the alkane structure. This means that the two carbon atoms can, but not necessarily must be, adjacent to each other. An alkylene structure can be linear or branched and is saturated. In case the alkylene group comprises only one carbon atom, the alkylene group is a methylene group (-CH₂-) which is connected to the rest of the molecule by two single bonds. Preferably, alkylene groups according to the present invention comprise methylene, ethylene, *n*-propylene, isopropylene, *n*-butylene, *sec*-butylene, *tert*-butylene, *n*-pentylene, 1-methylbutylene, 2-methylbutylene, 3-methylbutylene, neopentylene, 1-ethylpropylene, *n*-hexylene, 1,1-dimethylpropylene, 1,2-dimethylpropylene, 1,2-dimethylpropylene, 1-methylpentylene, 2-methylpentylene, 3-methylpentylne, 4-methylpentylene, 1,1-dimethylbutylene, 1,2-dimethylbutylene, 1,3-dimethylbutylene, 2,2-dimethylbutylene, 2,3-dimethylbutylene, 3,3-dimethylbutylene, 1-ethylbutylene, 2-ethylbutylene, 1,1,2-trimethylpropylene, 1,2,2-trimethylpropylene, 1-ethyl-1-methylpropylene, 1-ethyl-2-methylpropylene, 1-ethyl-2-methylpropylene and the like. These structures can be limited in choice, in case the invention defines the carbon atoms of an alkylene group in a different manner. Moreover, the alkylene group according to the present invention optionally comprises at least one carbonyl-group, optionally comprises at least one halogen atom and/or optionally is interrupted by at least one heteroatom. Examples for such alkylene groups are -C(=O)-(CH₂)₄-C(=O)-, -C(=O)-(CH₂)₃-C(=O)-, -C(=O)-(CH₂)₂-C(=O)-, -C(CF₃)₂, -O-(CH₂)₄-O-, -O-(CH₂)₃-O-, -O-(CH₂)₂-O- or the like.

"Alkylidene" in the context of the present invention, for example and if not mentioned differently, refers to a bridging alkane structure of which two hydrogen atoms from the same carbon atom are removed. The alkylidene group optionally comprises at least one carbon-carbon-double bond, optionally comprises at least one carbonyl-group and/or optionally comprises at least one halogen atom. Preferably, alkylidene groups according to the present invention and/or in context with formula (3) comprise CH₂=C*, C(CH₃)₂=C*, isopropylidene, n-propylidene, isoheptylidene, C*(CH₃)(C(=O)CH₃), C(Cl₂)=C*, C(Br₂)=C* or the like, wherein the "C*" indicates the carbon atom which is at the position indicated as "Z" in formula (1).

"Cycloalkylene" in the context of the present invention, for example and if not mentioned differently, refers to a bridging cycloalkane structure of which two hydrogen atoms from different carbon atoms in the ring are removed. In this context the two carbon atoms of which the two hydrogens atoms are removed can be removed from any carbon atom which is present in the cycloalkane structure. This means that the two carbon atoms can, but not necessarily must be, adjacent to each other. According to the present invention the cycloaliphatic group of the cycloalkylene group is fused to at least one further cycloaliphatic ring. Examples of such a cycloalkylene group is the adamantanylene (tricyclo [3.3. 1.1 3,7 ] decanediyl).

"Cycloalkylidene" in the context of the present invention, for example and if not mentioned differently, refers to a bridging cycloalkane structure of which two hydrogen atoms from the same carbon atom in the ring are removed. The cycloaliphatic group of the cycloalkylidene group is optionally fused to at least cycloaliphatic and /or at least one aromatic ring. Examples of such cycloalkylidene groups are cyclopentylidene, cyclohexylidene, 3,3,5-trimethylcyclohexylidene, cyclodecylidene, cyclododecylidene, tetrahydrodicyclopentylidene, 9-fluorenylidene or the like.

"Aralkylidene" in the context of the present invention, for example and if not mentioned differently, refers in each case independently to a bridging straight-chain, cyclic, branched or unbranched alkyl structure of which two hydrogen atoms from the same carbon atom are removed and which is singly, multiply or polysubstituted by aryl radicals. In parallel, "aralkylene" refers in each case independently to a bridging straight-chain, cyclic, branched or unbranched alkyl structure of which two hydrogen atoms from the different carbon atoms are removed and which is singly, multiply or polysubstituted by aryl radicals. "Aryl" in the context of the present invention, for example and if not mentioned differently, is a carbocyclic aromatic radical. Examples of "aryl" are phenyl, *o-, p-, m*-tolyl, naphthyl, phenanthryl or anthracenyl. Examples of such aralkylidene groups especially in the context of formula (3) are phenyl-CH*, phenyl-C*(CH₃), naphthyl-CH*, phenyl-C*-phenyl or the like, wherein the "C*" indicates the carbon atom which is at the position indicated as "Z" in formula (1). Examples of such aralkylene groups are *m*-diisopropylidene phenylene, *p*-diisopropylidene phenylene.

"Alkoxy" in the context of the invention, for example and if not mentioned differently, refers to a linear, cyclic or branched alkyl group singularly bonded to oxygen (-OR). Preferably, alkoxy groups according to the present invention have 1 to 6 carbon atoms and, thus, comprise methoxy, ethoxy, *n*-propoxy, isopropoxy, *n*-butoxy, *sec*-butoxy, *tert*-butoxy, *n*-pentoxy, 1-methylbutoxy, 2-methylbutoxy, 3-methylbutoxy, neopentoxy, 1-ethylpropoxy, cyclohexoxy, cyclopentoxy, *n*-hexoxy, 1,1-dimethylpropoxy, 1,2-dimethylpropoxy, 1,2-dimethylpropoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy, 1-ethyl-2-methylpropoxy or 1-ethyl-2-methylpropoxy. These structures can be limited in choice, in case the invention defines the carbon atoms of an alkoxy group in a different manner.

A "halogen atom" in the context of the invention, if not mentioned differently, refers to fluorine (F), chlorine (Cl), bromine (Br), or iodine (I). Preferably, a halogen atom is F, Cl, Br or I, more preferably it is Cl or Br.

Based on the above-given definitions the skilled person knows how to understand further definitions which are not explicitly defined in the passage above.

Moreover, the inventive composition comprises more than 0 ppm and less than 1000 ppm of sodium. Preferably the inventive composition comprises 0.1 to 900 ppm, still preferably 1 ppm to 800 ppm, still preferably 5 ppm to 750 ppm, still preferably 8 ppm to 700 ppm, still preferably 10 ppm to 600 ppm, still preferably15 ppm to 500 ppm, still preferably 20 ppm to 400 ppm, still preferably 30 ppm to 300 ppm, still preferably 50 ppm to 200 ppm and most preferably 60 ppm to 100 ppm of sodium.

Preferably, the sodium content in the inventive composition is determined using inductively coupled plasma optical emission spectrometry.

According to the present invention, it was found that sodium influences the quality of the inventive composition. Especially, it has a negative impact on other polymeric component(s) and/or additive when the rubber-modified graft polymer containing sodium is compounded with other polymeric partner(s) and/or additives. In the present examples, it was found that if a rubber-modified graft polymer comprises too high amounts of sodium the polycarbonate with which it was blended degrades. This can be seen by the formation of free BPA and the decrease of the glass transition temperature of the polycarbonate in the blend. Thus, it can be seen that some amount of sodium is tolerable for the rubber-modified graft polymer to be useful in compound with other polymeric components and/or additives. However, the amount of sodium should not be too high in order to result in compositions having the desired mechanical and optical properties.

Furthermore, it is preferred that the inventive composition additionally comprises more than 0 ppm dimethyl carbonate. Still preferably, the inventive composition comprises 0.1 ppm to 1 000 ppm, still preferably 1 ppm to 900 ppm, still preferably 5 ppm to 800 ppm, still preferably 10 ppm to 600 ppm, still preferably 20 ppm to 500 ppm, still preferably 30 ppm to 400 ppm, still preferably 50 ppm to 200 ppm and most preferably 60 ppm to 100 ppm of dimethyl carbonate.

Preferably, the amount of dimethyl carbonate in the inventive composition is determined using gas chromatography.

Furthermore, it is preferred that the inventive composition additionally comprises less than 400 ppm vinyl monomer. Still preferably, the inventive composition comprises 0.0001 ppm to 300 ppm, still preferably 0.001 ppm to 200 ppm, still preferably 0.01 ppm to 100 ppm, still preferably 0.1 ppm to 100 ppm, still preferably 1 ppm to 50 ppm, still preferably 2 ppm to 20 ppm and most preferably 3 ppm to 10 ppm vinyl monomer.

Preferably, the vinyl monomer is selected from the group consisting of vinylaromatics and/or ring-substituted vinylaromatics (such as styrene, α-methylstyrene, p-methylstyrene, p-chlorostyrene), (C₁-C₂₀)-alkyl (meth)acrylates, preferably (C₁-C₈)-alkyl (meth)acrylates, such as methyl methacrylate, ethyl methacrylate, , n-butyl acrylate, t-butyl acrylate and/or vinyl cyanides (unsaturated nitriles such as acrylonitrile and methacrylonitrile). Most preferably, the vinyl monomer is styrene.

Within the scope of the present invention, ppb and ppm - unless otherwise specified - are to be understood as parts by weight.

The indicated ppm are based on the amount of the sum of B.1 and B.2. It is understood that if B.2 is not present in the inventive composition, the ppm are based on the amount B.1.

Preferably, the inventive composition comprises the indicated amounts, more preferably the preferred amounts of methanol, the at least one aromatic dihydroxy compound, preferably bisphenol A, and sodium in any combination of ranges. Still preferably, the inventive composition additionally comprises any of the indicated amounts, more preferably the preferred amounts of the vinyl monomer, preferably styrene and /or dimethyl carbonate in any combination of ranges.

More preferably, the inventive composition comprises
- 0.5 ppm to 9 000 ppm, preferably 5 ppm to 2 500 ppm, more preferably 10 ppm to 750 ppm and most preferably 15 ppm to 500 ppm of methanol,
- 1.5 ppm to 9 000 ppm, preferably 10 ppm to 5 000 ppm, more preferably 100 ppm to 3 000 ppm and most preferably 200 ppm to 2 000 ppm of the at least one aromatic dihydroxy compound, preferably bisphenol A,
- 0.1 to 900 ppm, preferably 15 ppm to 500 ppm, more preferably 30 ppm to 300 ppm and most preferably 60 ppm to 100 ppm of sodium,
- 0.1 ppm to 1 000 ppm, preferably 10 ppm to 600 ppm, more preferably 30 ppm to 400 ppm and most preferably 60 ppm to 100 ppm of dimethyl carbonate,
- 0.0001 ppm to 300 ppm, preferably 0.01 ppm to 100 ppm, more preferably 1 ppm to 50 ppm and most preferably 3 ppm to 10 ppm vinyl monomer, preferably styrene.

Preferably, the inventive composition comprises less than 100 ppm acetone, more preferably less than 50 ppm acetone, most preferably less than 5 ppm acetone.

The inventive compositions comprises a rubber-modified graft polymer B.1 and optionally at least one vinyl(co)polymer B.2. Preferably, the inventive compositions comprises a rubber-modified graft polymer B.1 and at least one vinyl(co)polymer B.2.

Preferably, the rubber-modified polymer B.1 comprises
B.1.1 5 to 95% by weight, preferably 20 to 92% by weight, in particular 30 to 91% by weight, based on the graft polymer, of at least one vinyl monomer on
B.1.2 95 to 5% by weight, preferably 80 to 8% by weight, in particular 70 to 9% by weight, based on the graft polymer, of one or more rubber-elastic graft substrates having glass transition temperatures < -10°C, more preferably < -40°C, particularly preferably < - 70°C, determined by dynamic scanning calorimetry (DSC) according to DIN EN 61006 in the version of 2004 at a heating rate of 10 K/min with determination of Tg as the midpoint temperature (tangent method).

The graft substrate B.1.2 preferably has a median particle size (D50) of 0.05 to 10.00 µm, preferably of 0.1 to 5.0 µm, and particularly preferably of 0.1 to 1.5 µm.

The median particle size D50 is the diameter with 50 % by weight of the particles above it and 50 % by weight below it. Unless expressly stated otherwise in the present invention it is determined for all components by means of ultracentrifuge measurement (e. g. W. Scholtan, H. Lange, Kolloid, Z. und Z. Polymere [Polymers] 250 (1972), 782-1796).

The monomers B.1.1 are preferably mixtures of
B.1.1.1 65 to 85 % by weight, particularly preferably 70 to 80 % by weight, more preferably 74 to 78 % by weight, in each case based on the sum of B.1.1.1 and B.1.2.1, of vinylaromatics and/or ring-substituted vinylaromatics (such as styrene, α-methylstyrene, p-methylstyrene, p-chlorostyrene) and/or (C₁-C₂₀)-alkyl (meth)acrylates, preferably (C₁-C₈)-alkyl (meth)acrylates, such as methyl methacrylate, ethyl methacrylate, and
B.1.2.1 15 to 35 % by weight, particularly preferably 20 to 30 % by weight, more preferably 22 to 26 % by weight, in each case based on the sum of B.1.1.1 and B.1.2.1, of vinyl cyanides (unsaturated nitriles such as acrylonitrile and methacrylonitrile) and/or (C₁-C₂₀)-alkyl (meth)acrylates, preferably (C₁-C₈)-alkyl (meth)acrylates, such as methyl methacrylate, n-butyl acrylate, t-butyl acrylate, and/or derivates (such as anhydrides and imides) of unsaturated carboxylic acids, for example maleic anhydride.

Preferred monomers B.1.1.1 are selected from at least one of the monomers styrene, α-methylstyrene and methyl methacrylate; preferred monomers B.1.2.1 are selected from at least one of the monomers acrylonitrile, maleic anhydride and methyl methacrylate. Particularly preferred monomers are B.1.1.1 styrene and B.1.2.1 acrylonitrile.

Suitable graft substrates B.1.2. of the graft polymers include for example diene rubbers, EP(D)M rubbers, i.e. those based on ethylene/propylene and optionally diene, acrylate, polyurethane, silicone, chloroprene, ethylene/vinyl acetate and also acrylate-silicone composite rubbers.

Preferred graft substrates B.1.2 are diene rubbers, preferably comprising butadiene or copolymers of dienes, preferably comprising butadiene, and further copolymerizable vinyl monomers (e.g. according to B.1.1.1 and B.1.2.1) or mixtures of one or more of the above-mentioned components.

A particularly preferred graft substrate B.1.2 is pure polybutadiene rubber. **In** a further preferred embodiment B.1.2 is styrene-butadiene rubber, particularly preferably styrene-butadiene block copolymer rubber.

Alternatively preferred graft substrates B.1.2 are acrylate rubbers, preferably selected from polymers of alkyl acrylates, optionally with up to 40 % by weight, based on B.1.2, of other polymerizable, ethylenically unsaturated monomers. Preferred alkyl acrylates include C₁ to C₈ alkyl esters, especially methyl, ethyl, butyl, n-octyl and 2-ethylhexyl esters; halogenated alkyl esters, preferably halogenated C₁ to C₈ alkyl esters, such as chloroethyl acrylate, and mixtures of these monomers. Preferred 'other' polymerizable, ethylenically unsaturated monomers which, in addition to the alkyl acrylates, can optionally be used to produce the graft substrate B.1.2 are, for example, acrylonitrile, styrene, *-methyl styrene, acrylamides, vinyl C₁ to C₆ alkyl ether, methyl methacrylate, butadiene.

The gel fraction of the graft substrate B.1.2 is at least 30 % by weight, preferably at least 40 % by weight, in particular at least 60 % by weight, in each case based on B.1.2 and measured as insoluble fraction in toluene.

The gel content of the graft substrate B.1.2 is determined at 25 °C in a suitable solvent as content insoluble in these solvents (M. Hoffmann, H. Krömer, R. Kuhn, Polymeranalytik I und II, Georg Thieme-Verlag, Stuttgart 1977).

Most preferred as rubber-modified graft polymer is acrylonitrile-butadiene-styrene (ABS).

The graft copolymers in component B.1 are produced by free-radical polymerization, for example by emulsion, suspension, solution or bulk polymerization. Mixtures of graft polymers produced in different processes may also be used as component B.1. The graft polymers in component B.1 are preferably produced by emulsion or bulk polymerization.

Suitable graft polymers produced in the emulsion polymerization process are for example ABS polymers produced in the emulsion polymerization process by redox initiation with an initiator system composed of organic hydroperoxide and ascorbic acid according to US-P 4 937 285.

Further suitable graft polymers produced in the emulsion polymerization process are MBS modifiers having a core-shell structure.

Suitable polymers according to component B) prepared by bulk polymerization are described for example in DE-OS 2 035 390 (=US PS 3 644 574) or in DE-OS 2 248 242 (=GB-PS 1 409 275), or in Ullmanns, Enzyklopädie der Technischen Chemie, Vol. 19 (1980), p. 280 et seq.

Most preferably, in the inventive composition the rubber-modified graft polymer B.1 comprises
a first rubber-modified graft polymer B.1-A obtained by emulsion polymerization and
a second rubber-modified graft polymer B.1-B obtained by bulk polymerization.

This is especially true if the inventive composition is a recycled composition. Furthermore, it is true if the inventive composition is obtained from an end-of-life material. Such materials typically are mixtures of various compounds and therefore, contain different rubber-modified graft polymers.

The rubber-modified graft polymer may comprise free, i.e. not chemically bonded to the rubber substrate and not included in the rubber particles, vinyl(co)polymer. This vinyl(co)polymer is referred to as optional vinyl(co)polymer B.2. Typically, a rubber-modified graft polymer in addition comprises at least one (rubber-free) vinyl(co)polymer B.2. The vinyl(co)polymer B.2 preferably comprises structural units derived from the vinyl monomers according to B.1.1 above. Preferably, the vinyl(co)polymer comprises structural units derived from at least one vinyl monomer selected from the group consisting of vinylaromatics, ring-substituted vinylaromatics, (C1-C8)-alkyl (meth)acrylates, vinyl cyanides and unsaturated carboxylic acids. More preferably, the vinyl(co)polymer B.1 comprises structural units derived from at least one vinyl monomer selected from the group consisting of vinylaromatics, (C1-C8)-alkyl (meth)acrylates and vinyl cyanides. It is understood that also combinations of the mentioned vinyl monomers are encompassed.

Such vinyl(co)polymer B.1 may be formed in the rubber-modified graft polymer during the polymerization of the graft polymers owing to the method of production (the grafting on the graft substrate is not necessarily complete) or may else be polymerized and admixed with the rubber-modified graft polymer separately. It is likewise possible for a portion of the free vinyl(co)polymer B.1 in the rubber-modified graft polymer to originate from the graft polymer itself owing to the method of production and for another portion to be polymerized and admixed with the rubber-modified graft polymer separately.

Preferably, this vinyl(co)polymer B.1 has in the rubber-modified graft polymer a weight-average molecular weight M_{w} of 30 to 250 kg/mol, preferably of 70 to 200 kg/mol, in particular of 90 to 180 kg/mol (measured by gel permeation chromatography (GPC) in tetrahydrofuran against a polystyrene standard). Preferably and in particular preferably in the context of the given weight-average molecular weight above the vinyl(co)polymer is styrene acrylonitrile resin (also known as SAN).

**In** the context of the present invention, the weight average molecular weight Mw of the vinyl(co)polymer B.1 in the rubber-modified graft polymer is measured by gel permeation chromatography (GPC) in tetrahydrofuran against a polystyrene standard.

The inventive composition can be a composition which comprises recycled rubber-modified graft polymer and optionally vinyl(co)polymer. In this case the vinyl(co)polymer can be recycled, too. The inventive composition can be a mixture of recycled rubber-modified graft polymer, optionally recycled vinyl(co)polymer and virgin rubber-modified graft polymer and optionally virgin vinyl(co)polymer.

Preferably, the inventive composition has a particle size D50 of 20 to 1000 µm, more preferably of 50 to 200 µm and most preferably of 80 to 150 µm. Preferably, the particle size is determined using dynamic light scattering (DLS) in water as solvent.

More preferably, the inventive composition is obtainable or obtained by a recycling process of a composition comprising a polycarbonate and a rubber-modified graft polymer B.1. This composition can comprise at least one vinyl(co)polymer, too. Most preferably, the recycling process is a methanolysis.

According to the present invention, it is understood that the inventive composition comprises positive amounts of methanol, at least one aromatic dihydroxy compound and sodium. These components intrinsically can remain in the inventive composition due to the fact that a recycling process, preferably a methanolysis is used in order to obtain the inventive composition.

It was found that when using a specific process of methanolysis which is described below, a composition comprising a rubber-modified graft polymer and optionally a vinyl(co)polymer can be obtained which has positive amounts of methanol, at least one aromatic dihydroxy compound and sodium, but still has high quality. Especially, it can be used in subsequent compounding processes with a negative impact on the polymeric partner and/or additive it is compounded with. Thus, the inventive composition which results from the specified methanolysis, wherein the rubber-modified graft polymer is in the liquid phase at least at the end of the methanolysis reaction, has positive amounts of educts, products and catalyst, however, it maintains its basic properties and the positive amounts are in an acceptable range so as to provide a composition which could replace virgin material of the same composition.

Especially, it was found that the use of the specific process of a methanolysis of an article comprising polycarbonate and a rubber-modified graft polymer B.1 and optionally a vinyl(co)polymer B.2 using specific ratios of methanol and optionally dimethyl carbonate with respect to the rubber-modified graft polymer B.1 and optionally vinyl(co)polymer B.2 leads to an efficient and scalable recycling process. In particular the rubber-modified graft polymer B.2 and optionally the one vinyl(co)polymer B.2 could be recycled. This leads to an economically and/or ecologically efficient process. The specific ratios of the components are chosen in a way so that as few resources (e. g. energy and/or (raw) materials such as catalyst, reactants and/or solvents, e. g. methanol and/or dimethyl carbonate) as possible are needed. Moreover, this also requires smaller vessels or provides the possibility of recycling larger quantities due to high concentrations (especially when compared to a reaction having the same reaction time).

According to the present invention, the ratios of the components in the reaction mixture of the methanolysis of an article comprising polycarbonate comprising a structural unit derived from an aromatic dihydroxy compound and a rubber-modified graft polymer B.2 and optionally a vinyl(co)polymer B.2 are chosen in a way so that the rubber-modified graft polymer B.1 and optionally the vinyl(co)polymer B.2 are completely in the liquid phase (at least at the end of the methanolysis reaction of step (ii)). This results in a viscosity of the reaction product (comprising at least the aromatic dihydroxy compound and the rubber-modified graft polymer) which is easily processable. The process parameters prevent the formation of a sticky vinyl(co)polymer/rubber-modified graft polymer residue and at the same time allow the efficient depolymerization of PC and recovery of its monomers. It was found that following the methanolysis processes of the prior art where the rubber-modified graft polymer either is a sticky coating or a solid, the aromatic dihydroxy compound sticks to the rubber-modified graft polymer. Even when washing, this aromatic dihydroxy compound cannot be easily removed from the rubber-modified graft polymer. This means that a high number of washing steps is required in order result in a composition comprising the rubber-modified graft polymer with acceptable quality. Moreover, it was found that the aromatic dihydroxy compound cannot be fully removed from the rubber-modified graft polymer (cf. examples). However, when using the process according to the present invention the aromatic dihydroxy compound can be recovered (e.g. washed) from the rubber-modified graft polymer more easily. This means that less washing steps are needed to get an economically reasonable process and result in a composition comprising the rubber-modified graft polymer with high quality. This is especially true when compared to a process where the rubber-modified graft polymer is not completely in the liquid phase at the end of the methanolysis reaction. Moreover, this means that a high quality of the inventive composition can be obtained using less solvent for washing and / or less purification steps resulting in less energy consumption. Moreover, the resulting at inventive composition comprises less aromatic dihydroxy compounds and sodium especially when referring to the same effort of washing.

Moreover, the process of the present invention was found to be scalable and at the same time ecologically and/or economically effective. It was found that the rubber-modified graft polymer B.1 and optionally the vinyl(co)polymer B.2 can be precipitated in a way that it is filterable and can be easily removed from the (other) reaction products. This filtration needs low pressures and is very quick.

The process of the present invention is for the recovery of a rubber-modified graft polymer B.1 and optionally a vinyl(co)polymer B.2 from an article comprising the steps of
(i) bringing into contact at least an article, methanol, a catalyst and optionally dimethyl carbonate to form a reaction mixture, wherein the article comprises
   (A) polycarbonate comprising a structural unit derived from an aromatic dihydroxy compound and
   (B) a rubber-modified graft polymer and optionally at least one vinyl(co)polymer,
(ii) performing a methanolysis reaction of the reaction mixture of step (i) yielding at least the aromatic dihydroxy compound from component A),
   characterized in that the amounts of methanol, component A), component B) and the optionally present dimethyl carbonate before step (ii) are chosen so that the following conditions (a) to (c) are met at the end of step (ii), wherein the conditions (a) to (c) represent weight ratios which are obtained using the assumption that the methanolysis reaction of component A) is theoretically completed to 100 %:
   (a) the weight ratio of dimethyl carbonate : component B) is in the range of 0.5 : 1 to 20 : 1,
   (b) the weight ratio of methanol : component B) is in the range of 0.79 : 1 to 4 : 1 and
   (c) the weight ratio of (methanol + dimethyl carbonate) : (component B) + aromatic dihydroxy compound) is in the range of 0.6 : 1 to 20 : 1.

The process provides the rubber-modified graft polymer B.1 and optionally the at least one vinyl(co)polymer as direct product of the process. It also provides an aromatic dihydroxy compound as direct product of the process.

The aromatic dihydroxy compound is obtained by the methanolysis of the component A) of the article. The skilled person knows how aromatic dihydroxy compounds can be used in order to obtain a polycarbonate and thus, it is also known to him how a polycarbonate is "derived" from an aromatic dihydroxy compound. Typically, those compounds are linked with carbonate groups while each of the two hydrogens is removed and the remaining oxygen is part of the carbonate group. This carbonate group of the polycarbonate is cleaved once again in the methanolysis reaction so that in the end the aromatic dihydroxy compound is formed (once again). This aromatic dihydroxy compound can be referred to as building block or monomer of the polycarbonate. In this context the term "structural unit" is used according to the invention. It preferably refers to a structural unit derived from a monomer formed by polymerizing a monomer, or a structural unit in which a portion of the structural unit is converted to another structure by processing a polymer. Preferably, the structural unit of the polycarbonate is represented by the above given description of the aromatic dihydroxy compound, wherein the two hydrogens are removed and which are linked to each other by a carbonate group (cf. above). Accordingly, the skilled person is capable of deriving a structural unit of the polycarbonate when seeing the aromatic dihydroxy compound. Moreover, it is understood by the skilled person that the aromatic hydroxy compound is organic.

### Component A)

As already described above, the skilled person is capable of identifying the connection between the polycarbonate which is used as component A) and the aromatic dihydroxy compound as recovered according to the inventive process. In the context of the present invention the term "polycarbonate" refers to a polymer that contains multiple carbonate groups. These carbonate groups are incorporated into the polymer backbone. This means that a polycarbonate preferably has repeating units of the form ...-(R-O-(C=O)-O)-...

According to the present invention in the process an article is used which comprises a polycarbonate comprising a structural unit derived from an aromatic dihydroxy compound as component A) (e. g. HO-R-OH, wherein the R represents the R as given for the above-shown repeating units of the polycarbonate). This polycarbonate is degraded by the process of the present invention to give the aromatic dihydroxy compound. Accordingly, the skilled person can understand the structure of the polycarbonate based on the structure as given for the aromatic dihydroxy compound. As example, the skilled person knows the structure of bisphenol A based polycarbonate.

This means that "polycarbonates" in the context of the present invention generally comprise other structures besides the carbonate base structures. It is also possible that they comprise even other structures besides the carbonate base structures and the structural units which are derived from the aromatic dihydroxy compound. For example, typically a polycarbonate comprises a chain terminator. Moreover, this is can be especially true in case the article which comprises component A) is post-industrial waste or post-consumer waste, also known as end-of-life (EoL) material. Those materials might have structures which can be called "defective structures" due to the lifecycle of the polycarbonate. However, such "defective structures" can also be present in the virgin polycarbonate. Their amount could be higher than the amount in virgin polycarbonate. Polycarbonate chains are known to degrade under harsh conditions and/or UV light leading to rearrangement structures etc. Those structures are known to the skilled person. The presence of such structures diverging from the pure base structure does not depart from the scope of the present invention. Especially when referring to such EoL materials the exact structure of the polycarbonate might be unknown. However, the skilled person is able to determine whether the polycarbonate is derived from an aromatic dihydroxy compound (and in most cases also which exact compound). This is especially true as most of those materials comprise polycarbonate derived from bisphenol A.

According to the present invention, when calculating the weight of the aromatic dihydroxy compound to be obtained (cf. condition (c) described in more detail below), the exact structure and /or molecular weight of the polycarbonate in the article is preferably not considered. The chemical nature of the aromatic dihydroxy compound is determined. This can be done by methods known to the skilled person. Preferably, the chemical nature of the polycarbonate can be determined by IR-spectroscopy and/or NMR-spectroscopy. It is also possible to firstly depolymerize the polymer (e. g. by saponification as described below) and to conduct a gas chromatography. Those methods are known to the skilled person. For example, when using IR spectroscopy, a KBr pellet of the article can be analyzed and compared to a calibration. By this the polycarbonate content can be determined and the amount of the aromatic dihydroxy compound could be calculated, for example by using the formula: $\begin{matrix}\frac{m \left(polycarbonate\right)}{molecular weigth of structural unit of PC} ∗ molecular weight of monomer = \\ m \left(monomer\right) ,\end{matrix}$ wherein m(polycarbonate) represents the mass of the polycarbonate, the molecular weight of the monomer is the structural unit of PC, wherein C=O is substracted and two Hs are added (resulting in two OH-groups and no carbonate groups) and the molecular weight of the structural unit of PC includes a carbonate group (and no OH-groups). It is also possible to determine the weight of the aromatic dihydroxy compound to be obtained by saponification of the article and thus, the polycarbonate. This leads to the full degradation of the polymer chain to its monomers. This method might be more appropriate in case the IR spectrum of the article itself has significant overlaps of signals.

For example, the article could be subjected to a total hydrolysis to form the corresponding degradation products. This may be accomplished for example as follows: The article is hydrolyzed under reflux by means of sodium methoxide (for example in dichloromethane). The corresponding solution is acidified (for example by HCl) and concentrated to dryness. The drying residue is dissolved in acetonitrile and the aromatic dihydroxy compounds are determined for example by means of HPLC with UV detection.

The article and the monomers are preferably analyzed by IR spectroscopy. Based on the results of such IR analysis, the weight of the aromatic dihydroxy compound can be calculated as follows $\frac{molecular weight of monomer}{molecular weigth of structural unit of PC} ∗ 100 = % monomer in PC$ wherein the molecular weight of the monomer is the structural unit of PC, wherein C=O is substracted and two Hs are added (resulting in two OH-groups and no carbonate groups) and the molecular weight of the structural unit of PC includes a carbonate group (and no OH-groups). Knowing the amount of PC to be depolymerized multiplied with the % of monomer in PC results in the weight of the aromatic dihydroxy compound which is then used in condition (c).

Using bisphenol A (BPA) as example, the weight of BPA which is theoretically obtained by the inventive process assuming 100 % conversion is: $\frac{228 \frac{g}{mol}}{254 \frac{g}{mol}} ∗ 100 = 89.76 % BPA in PC$

When calculating the weight of the aromatic dihydroxy compound to be obtained (cf. condition (c) described in more detail below), all of the other structures which might be present in the polycarbonate are ignored. This is especially true for the chain terminator and/or any structure formed by rearrangement reactions or side reactions. Polycarbonates according to component A) which are suitable according to the invention are known from the literature or can be produced by processes known from the literature (for production of polycarbonates see by way of example Schnell, "Chemistry and Physics of Polycarbonates", Interscience Publishers, 1964 and also DE-AS (German Published Specification) 1 495 626, DE-A 2 232 877, DE-A 2 703 376, DE-A 2 714 544, DE-A 3 000 610, DE-A 3 832 396).

Aromatic polycarbonates are produced by way of example by reaction of diphenols with carbonyl halides, preferably phosgene and/or with aromatic diacyl dihalides, preferably dihalides of benzenedicarboxylic acids, by the interfacial process, optionally using chain terminators, for example monophenols, and optionally using trifunctional or more than trifunctional branching agents, for example triphenols or tetraphenols. Production via a melt polymerization process by reaction of diphenols with, for example, diphenyl carbonate is likewise possible.

Preferably, the polycarbonate of component A) comprises at least one structural unit of formula (3) to (10) 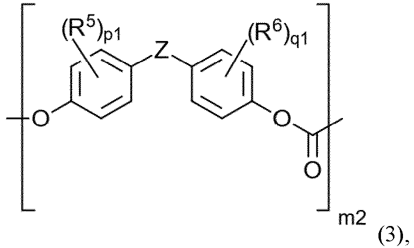 wherein each Z each R^{5,} R⁶ and each of p1 and q1 have the meanings as given above for formula (1) and m2 represents the average number of repeating units, 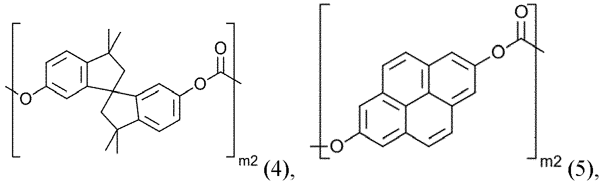 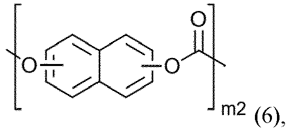 wherein in formula (4) to (6) m2 represents the average number of repeating units; 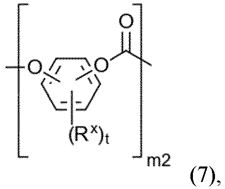 wherein each R^{x} independently represents linear or branched C₁-C₆-alkyl, C₁-C₁₂-aryl, C₁-C₈-aralkyl or a halogen atom, t is 0 to 4 and m2 represents the average number of repeating units; 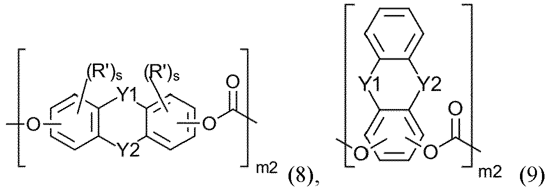 wherein in formula (8) and (9) each Y1 independently represents oxygen, sulfur or N-R^{y}, wherein R^{y} is H or -CH₃, each Y2 interpedently represents a single bond, oxygen, sulfur or N-R^{y} as defined above and m2 represents the average number of repeating units and in formula (8) each R' independently represents a linear C₁-C₄-alkyl, branched C₃-C₄ alkyl, aralkyl or aryl and s is 0 to 2; 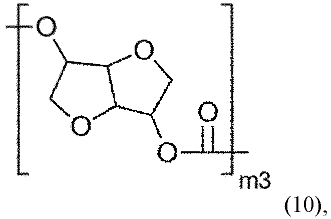 wherein m3 represents the average number of repeating units.

Moreover, all preferred meanings and combinations of preferred meanings with respect to above-given formula (1) apply. In accordance with the above-given meanings for formula (1), the polycarbonate of component A) most preferably comprises structural units represented by formula (2a) and/or (2b) 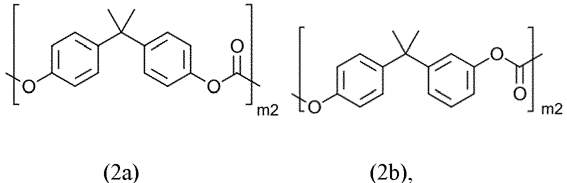 wherein m2 represents the average number of repeating units, preferably 6 to 60.

Moreover, the polycarbonate of component A) preferably comprises at least one structural unit of formula (3) above, most preferably of formula (2a), and a structure of formula (2c) 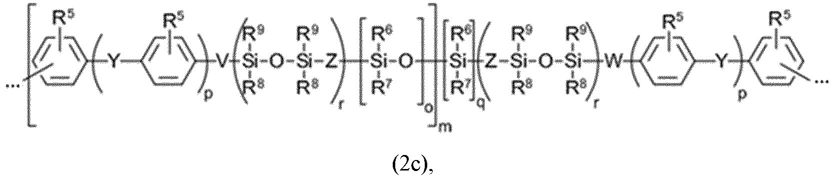 in which
R⁵ represents hydrogen or C₁ to C₄ alkyl, C₁ to C₄ alkoxy, preferably hydrogen or methyl or methoxy, particularly preferably hydrogen,
R⁶, R⁷, R⁸ and R⁹ mutually independently represents C₆ to C₁₂ aryl or C₁ to C₄ alkyl, preferably phenyl or methyl, in particular for methyl,
Y represents a single bond, -SO₂-, -S-, -CO-, -O-, C₁ to C₆ alkylene, C₂ to C₅ alkylidene, C₆ to C₁₂ arylene, which optionally can be condensed to at least one further aromatic ring, which further aromatic ring can comprise at least one hetero atom, or a C₅ to C₆ cycloalkylidene residue, which can be singly or multiply substituted with C₁ to C₄ alkyl, preferably a single bond, -O-, isopropylidene or a C₅ to C₆ cycloalkylidene residue, which can be singly or multiply substituted with C₁ to C₄ alkyl, and in particular isopropylidene,
V represents oxygen, C₂ to C₆ alkylene or C₃ to C₆ alkylidene, preferably oxygen or C₃ alkylene,
p, q and r mutually independently each stand 0 or 1,
with the provisio that if q = 0, W represents a single bond, if q = 1 and r = 0, W represents oxygen, C₂ to C₆ alkylene or C₃ to C₆ alkylidene, preferably oxygen or C₃ alkylene,
if q = 1 and r = 1, W and V each independently represent C₂ to C₆ alkylene or C₃ to C₆ alkylidene, preferably C₃ alkylene,
Z represents C₁ to C₆ alkylene, preferably C₂ alkylene,
o represents an average number of repeating units from 10 to 500, preferably 10 to 100,
m represents an average number of repeating units from 1 to 10, preferably 1 to 6, particularly preferably 1.5 to 5 and "..." represents the sites at which the structure of formula (2c) is incorporated into the polycarbonate.

The term "average number of repeating units" is known by the skilled person. The skilled person knows how to determine this parameter. Typically, it is determined by using a GPC method. Formulae (2a) and / or (2b) recite "m2" as number of repeating units. Preferably, m2 is 6 to 60, more preferably it is 10 to 55, even more preferably it is 20 to 50, still more preferably it is 25 to 45 and most preferably it is 30 to 40. Preferably, it is determined using the GPC method as outlined in the context of the present invention. Accordingly, according to the present invention it is preferred that the polycarbonate of component A) has a weight average molecular weight of 15000 g/mol to 40000 g/mol, more preferably 16000 g/mol to 34000 g/mol, still more preferably 17000 g/mol to 33000 g/mol and most preferably 19000 g/mol to 32000 g/mol as determined *via* size exclusion chromatography. Preferably, this size exclusion chromatography is calibrated using a bisphenol A polycarbonate and dichloromethane as eluent. Typically, linear polycarbonate (obtained from BPA and phosgene) having a known molecular weight distribution of PSS Polymer Standards Service GmbH, Deutschland can be used for the calibration. Most preferably, the calibration is performed according to method 2301-0257502-09D method (from 2009 in German) from the company Currenta GmbH & Co. OHG, Leverkusen. The eluent is dichloromethane. Preferably, a combination of columns based on crosslinked styrene divinylbenzene resins is used. Still preferably, the diameter of the analytical columns is 7,5 mm, length of 300 mm. The particle size of the column material is preferably 3 µm to 20 µm. The concentration of the solution is preferably 0.2 wt.-%. The flow rate is preferably 1.0 ml/min. The temperature of the solution is preferably 30 °C. Preferably, a UV- and/or RI-detection is used.

According to the present invention component A) can comprise more than one polycarbonate. This means that component A) can be a mixture of at least two polycarbonates. However, especially when referring to EoL materials this cannot easily be determined. As outlined above, the knowledge of such a fact is not detrimental in order to carry out the process of the present invention.

### Component B)

According to the present invention, the article comprises at least a rubber-modified graft polymer and optionally at least one vinyl(co)polymer as component B). According to the present invention the vinyl(co)polymer can be also referred to as "rubber-free vinyl(co)polymer". This especially helps to distinguish the rubber-modified graft polymer and the vinyl(co)polymer which is rubber free. The rubber-modified graft polymer and optionally at least one vinyl(co)polymer are preferably those as described above with respect to B.1 and B.2. Due to the methanolysis process some properties of the rubber-modified graft polymer and optionally at least one vinyl(co)polymer might change. However, the chemical natures as describe above remain essentially the same throughout the recycling process. Moreover, it is an aim of the present invention that the inventive composition comprising B.1 and optionally B.2 maintains its basic properties, e. g. glass transition temperature. Accordingly, it is obvious that the rubber-modified graft polymer and optionally at least one vinyl(co)polymer which are used as component B) preferably are the same as B.1 and B.2.

As described above with respect to component A), in EoL materials the chemical nature and/or the amount of component B) might be unknown. In order to determine the amount of component B) in the article, it is possible to depolymerize the article (e. g. saponify it as described above) and to determine the amount of the residue. It is also possible to analyze this residue to determine the chemical nature of component B). As described below, all unknown components in the article are attributed to component B) for the sake of calculating inventive conditions (a) to (c). Therefore, the determination of the chemical nature of those components is not required for carrying out the present invention.

### Article

According to the present invention an "article" is used from which the aromatic dihydroxy compound and the rubber-modified graft polymer B.1 and optionally the at least one vinyl(co)polymer B.2 is or are recovered. The article comprises components A) and B) as described above. Typically the article comprises a blend of at least component A) and B). However, it might be also possible that the article comprises component A) and component B) which are spatially separated, for example at least two layers, one layer of component A) and one layer of component B). Moreover, the article may comprise further components and/or layers in addition to component A) and B). Therefore, the article can comprise a composition comprising at least component A) and B). In this case the article may comprise further layers such as coatings or the article is a multilayer article. Finally, the article may be a mixture of different articles which comprises components A) and B) (e. g. a shredded article comprising component A) is mixed with a shredded article comprising B) to result in a mixed article comprising component A) and B). This may be encompassed when using the expression "the article comprises components A) and B)".

An "article" according to the present invention preferably means an article produced by processing a thermoplastic composition comprising at least component A) and B) into a (shaped or molded) part. Processing methods are known to the skilled person and include any plasticization method commonly used for polycarbonate, e.g. granulation, injection molding, (co)extrusion, blow molding, thermoforming. The article can have many different shapes and sizes, e.g. granules, pellets, injection-molded parts, sheets, films. Preferably, the article originates from post-industrial waste or post-consumer waste. This means that preferably, it is an end-of-life (EoL) material.

According to the present invention sometimes the term "polycarbonate blend composition" or "polycarbonate composition" is used. This refers to a composition comprising at least component A) and B).

The article according to the present invention comprises A) polycarbonate comprising a structural unit derived from an aromatic dihydroxy compound and B) a rubber-modified graft polymer and optionally at least one vinyl(co)polymer. The polycarbonate preferably comprises structural units derived from the aromatic dihydroxy compound of formula (1), more preferably from the aromatic dihydroxy compound of formulae (1a) or (1b), most preferably from the aromatic dihydroxy compound of formula (1a).

Preferably and most preferably in this context, component B) comprises as vinyl(co)polymer styrene-acrylonitrile copolymer and/or as rubber-modified graft polymer acrylonitrile-butadiene-styrene.

The article may optionally comprise one or more polymer additives or polymeric components C). Here, it is preferable that the composition forming the article comprising A) and B) comprise one or more polymer additives or polymeric components C). It is understood that in case the polymeric components C) are present, they are no polycarbonate, no vinyl(co)polymers and/or no rubber-modified graft polymers. Preferably, component C) is selected from the group consisting of flame retardants, anti-drip agents, flame retardant synergists, smoke inhibitors, lubricants and demolding agents, nucleating agents, antistatic agents, conductivity additives, stabilizers (e.g. heat stabilizers, hydrolysis and heat aging stabilizers, UV stabilizers and also transesterification inhibitors), flow promoters, phase compatibilizers, further polymeric constituents other than components A) and B) (for example functional blend partners), fillers and reinforcing agents, light diffusing agents, dyes and pigments. The article may optionally comprise one or more layers selected from the group consisting of coating layers, primer layers and co-extruded layers. This is especially the case when the article comprises a composition comprising at least component A) and B).

Preferably, component C) is selected from the group consisting of thermal stabilizers, antioxidants, UV absorbers, lubricants and mold release agents.

In the context of the present invention component C), if present, is ignored when calculating conditions (a) to (c). This means that for the calculation of (a) to (c) it is assumed that the article consists of component A) and component B), whereas all unknown components are attributed to the amount of B).

Preferably, the article consists of at least 75 wt.-%, more preferably at least 80 wt.-%, still preferably at least 85 wt.-%, still preferably at least 90 wt.-%, still preferably at least 95 wt.-% and most preferably at least 98 wt.-% of component A), B) and optionally C), based on the weight of the whole article.

In the context of the present invention, "article" can mean a single article or a mixture of articles. A mixture of articles may include articles of the same origin, e.g. type of application and/or type of material or material mixture, or articles of multiple different origins. Even if articles of the same origin are present, they may still differ in terms of material composition and/or quality.

It may be advantageous to reduce the particle size of the article by processes known to the skilled person such as shredding, crushing, cutting or milling prior to (bringing the article into contact with methanol, a catalyst and optionally dimethyl carbonate according to) step (i) of the process according to the present invention. A smaller particle size may increase the surface and also the depolymerization rate. Preferably, the average particle size of the article is below 1 cm, more preferably below 0.5 cm. This particle size preferably refers to the largest dimension of the shredded article. Within the context of the present invention all measures to reduce the size of the collected molded parts are preferably referred to as shredding and the articles obtained after shredding are referred to as shredded articles. Most preferably, inventive process step (i) comprises bringing into contact at least a shredded article, methanol, a catalyst and optionally dimethyl carbonate to form a reaction mixture.

### Catalyst

The methanolysis reaction is carried out in the presence of a catalyst. This is understood as "at least one catalyst". Thus, it is also feasible to employ mixtures of different catalysts. However, at least one catalyst comprises sodium.

Preferably, the at least one catalyst is a transesterification catalyst comprising sodium. Preferably, the catalyst is a base comprising sodium.

The catalyst is preferably selected from the group consisting sodium hydroxide, sodium carbonate, sodium hydrogen carbonate, sodium methoxide, sodium ethoxide, sodium oxide and sodium hydroxide. Most preferably, the catalyst is sodium hydroxide. This catalyst was found to provide sufficiently high depolymerization rates while being economically affordable.

The catalyst can be added as such or in solution. For example, sodium hydroxide can be added in water and/or methanol. Most preferably, the catalyst sodium hydroxide is added in water.

Preferably, the catalyst is used in an amount of 0.01 to 10 wt.-%, preferably 0.05 to 5 wt.-%, still preferably 0.1 to 1 wt.-% and most preferably 0.2 to 0.5 wt.-% with respect to component A). This amount of the catalyst results in an inventive composition having the defined amounts of sodium.

### Process step (i)

According to the present invention in process step (i) at least an article, methanol, a catalyst and optionally dimethyl carbonate are brought into contact to form a reaction mixture. The article and the catalyst are described above.

According to the present invention, the term "bringing into contact" is used. This is intended to encompass both embodiments in which one component/mixture etc. is added to another component/mixture etc., as well as the reverse order. How such contact between components and/or mixtures is established is known to those skilled in the art. This can be achieved, for example, by adding a component and/or mixture, introducing a component and/or mixture and afterwards adding another component and/or mixture, mixing a component and/or mixture, dripping in a component and/or mixture, etc. It is apparent to those skilled in the art that the term "adding," as occasionally used in the invention, can be understood as synonymous with "bringing into contact." The "bringing into contact in step (i) is preferably carried out with introduction of mixing energy. This may be carried out by methods known to those skilled in the art. It is known that increasing surface renewal influences the rate of methanolysis.

Moreover, according to the present invention it may not be possible to clearly distinguish between process step (i) and (ii). The presence of the catalyst in process step (i) may immediately initiate the methanolysis reaction. However, this does not depart from the present invention. It is clear to the skilled person that most preferably, in case process step (i) and (ii) cannot be clearly distinguished the amounts of methanol, component A), component B) and the optionally present dimethyl carbonate in inventive step (i) are chosen so that conditions (a) to (c) are met at the end of step (ii). This means that "before step (ii)" preferably means in step (i).

Most preferably, in inventive step (i) at least a shredded article, methanol, at least one catalyst and optionally dimethyl carbonate are brought into contact. The amount of methanol which is used in inventive step (i) is determined by inventive conditions (b) and (c). Those are described below.

Moreover, the expression that dimethyl carbonate is "optionally" brought into contact with the article, methanol and the catalyst is understood by the skilled person. Inventive conditions (a) and (c) determine whether dimethyl carbonate is present already in process step (i) or whether it is solely (and/or additionally) formed during the methanolysis reaction in step (ii). Without being bound to a theory it was found according to the present invention that inventive conditions (a) to (c) which are further described below guarantee that component B) is in a liquid / dissolved state at the end of process step (ii). Dimethyl carbonate is a solvent for component B). This means that according to the present invention the expression "component B) is dissolved" or "in a dissolved state" is used. However, depending on the chemical nature of component B), an emulsion can be formed optionally in the presence of a dissolved component B) (the latter can be the case if component B) comprises at least two components). This is the reason why sometimes it is described that component B) is "in the liquid state". Preferably, this means that component B) is present in the mixture as obtained after step (ii) as an emulsion and/or in a dissolved state. The skilled person knows that an emulsion is a finely distributed mixture of two normally immiscible liquids without visible separation. Most preferably, the emulsion is stable. This preferably refers to a mixture of at least two immiscible liquids where one liquid is dispersed in the other in the form of fine droplets, and this dispersion remains uniform over time without phase separation. According to the present invention this is encompassed when referring to a "solvent". Especially in case when component B) is a mixture of ABS and SAN, it was found that SAN seems to be at least partly dissolved after step (ii) and the ABS is present as emulsion.

As dimethyl carbonate is a solvent for component B) it has the ability to dissolve and/or form an emulsion with component B). The methanolysis reaction leads to the formation of dimethyl carbonate. Depending on the amount of component B) in the article different amounts of dimethyl carbonate are needed to support the dissolution and/or formation of an emulsion of component B). Therefore, conditions (a) to (c) are inventively defined in a way to reflect this connection of the presence of component B) and dimethyl carbonate. Further connections and influences are described below when explaining conditions (a) to (c).

Preferably, in inventive step (i) no further solvent, especially no further solvent for component B) is present. More preferably, in inventive step (i) no 1,4-dioxane, methylenchloride, tetrahydrofuran, N-methyl pyrrolidone, dimethyl sulfoxide, 1,3-dimethyl-2-imidazolidinone, chloroform and/or chlorobenzene is present. Still more preferably, in inventive step (i) no 1,4-dioxane, chloroform and/or chlorobenzene is present. According to the present invention water can be present in process step (i) and/or (ii). This can be intentionally added, for example when the catalyst is firstly dissolved in water and then used in process step (i). Moreover, it is also possible that water is introduced in process step (i) by residual moisture in the article. Moreover, in case any solvent (e. g. methanol and/or dimethyl carbonate) is recycled from the inventive process to process step (i), there might be some water in process step (i). Preferably, the amount of water in process step (i) is less than 3 wt.-% with respect to the reaction mixture of step (i). Still preferably, the total amount of any further component present in the reaction mixture in step (i) beside the article, methanol, at least one catalyst, optionally dimethyl carbonate, optionally water and optionally any side product which is formedis at most 0.05 molar equivalent, more preferably at most 0.01 molar equivalent compared to 1 molar equivalent of component A). It is especially preferable that in this case the amount of water in process step (i) is less than 3 wt.-% with respect to the reaction mixture of step (i). The side product of the process could originate from component B) and/or component C). Especially in case of any recycling of e. g. a solvent to process step (i) those might accumulate. Still preferably, the reaction mixture of step (i) essentially consists of the article, methanol, at least one catalyst, optionally dimethyl carbonate, optionally water and optionally any side product of the process. It is understood by the skilled person that this does not exclude impurities of the starting materials. Most preferably, the reaction mixture of step (i) consists of the article, methanol, at least one catalyst, optionally dimethyl carbonate, optionally water and optionally any side product of the process. According to the present invention it is advantageous to have as few components in the reaction mixture of step (i) as possible. This leads to an easier work up after methanolysis. Moreover, substances that are not that easy to handle (e. g. 1,4-dioxane, chloroform and/or chlorobenzene) and/or are halogenated can be avoided.

### Process step (ii)

In process step (ii) a methanolysis reaction is performed. This process is known to the skilled person. According to the present invention "methanolysis" preferably refers to a process that depolymerizes polycarbonate to produce aromatic dihydroxy compounds and dimethyl carbonate by using methanol. In principle, this reaction is a transesterification. Methanol as alcohol transesterifies the carbonate group which is bound to the structural unit derived from the aromatic dihydroxy compound resulting in a methanol carbonate bond (and if performed twice in dimethyl carbonate) and a degraded polymer chain with a hydroxy end group (and if performed twice at a structural unit of the polycarbonate it results in the aromatic dihydroxy compound). By way of example, the following scheme shows the theoretical methanolysis reaction of bisphenol A based polycarbonate (e.g. the reaction which is aimed at, possible side reactions are not shown):

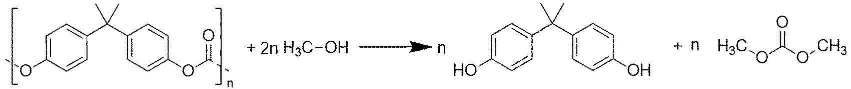

Therefore, the methanolysis reaction is capable of depolymerizing polycarbonate chains to yield at least the aromatic dihydroxy compound. The skilled person is aware that a copolycarbonate which comprises more than one structural unit yields more than one aromatic dihydroxy compound. Moreover, it was found that small amounts of water can be tolerated in this reaction and do not significantly influence the reaction as shown above.

Process step (ii) is preferably carried out in a temperature range of the reaction mixture from 50°C to 130 °C, preferably 60 °C to 120 °C, even more preferably 90 °C to 115 °C. Preferably, process step (ii) is conducted at a temperature which is above the glass transition temperature of component B). It was found that the overall reaction velocity significantly increases above the glass transition temperature of component B).

Preferably, the term "in a liquid state" at the end of inventive process step (ii) when referring to component B) refers to the temperature in which process step (ii) is conducted. More preferably, the term "in a liquid state" at the end of inventive process step (ii) when referring to component B) refers even to room temperature. This means that even when the mixture obtained by process step (ii) is cooled to room temperature, component B) preferably is still in the liquid phase.

Process step (ii) is preferably carried out in a pressure range from 1 bara to 15 bara, preferably 1.5 bara to 13 bara and most preferably 2 bara to 11 bara. Most preferably, process step (ii) is carried out in the above-given temperature range of the reaction mixture and at the above-given pressure range.

Process step (ii) is preferably carried out for 2 minutes to 20 hours, more preferably for 3 minutes to 5 hours, most preferred for 5 minutes to 1 hours. It is further preferred that the depolymerization of the polycarbonate is at least 95 % complete, further preferred at least 98 % complete, more preferred at least 99%, most preferred at least 99.5 %. A less complete depolymerization of polycarbonate leads to higher amounts of residual oligocarbonates in the product mixture at the end of process step (ii). This not only reduces the yield of the aromatic dihydroxy compound which can be recovered but also requires the removal of such oligocarbonates before recovering the aromatic dihydroxy compound and the a rubber-modified graft polymer B.1 and optionally the at least one vinyl(co)polymer B.2. It is therefore preferred that the polycarbonate conversion in the process according to the invention is as high as possible. It is understood that this actual yield is not the same as the theoretical yield of 100 % which underlies the calculation of conditions (a) to (c). This means that even if the methanolysis reaction is not carried out to 100 % conversion, the assumption that the methanolysis reaction of component A) is theoretically completed to 100 % for the calculation of conditions (a) to (c) still apply.

Preferably, process step (i) and/or process step (ii) are conducted under inert atmosphere (e. g. using nitrogen or argon).

### Conditions (a) to (c)

The process according to the present invention is characterized in that the amounts of methanol, component A), component B) and the optionally present dimethyl carbonate before step (ii) are chosen so that the following conditions (a) to (c) are met at the end of step (ii), wherein the conditions (a) to (c) represent weight ratios which are obtained using the assumption that the methanolysis reaction of component A) is theoretically completed to 100 %:
(a) the weight ratio of dimethyl carbonate : component B) is in the range of 0.5 : 1 to 20 : 1,
(b) the weight ratio of methanol : component B) is in the range of 0.79 : 1 to 4 : 1 and
(c) the weight ratio of (methanol + dimethyl carbonate) : (component B) + aromatic dihydroxy compound) is in the range of 0.6 : 1 to 20 : 1.

For the skilled person it is clear that component A) is not present as such in conditions (a) to (c). However, the amount of component A) determines how much aromatic dihydroxy compound can theoretically be formed by methanolysis. Therefore, as in condition (c) the amount of the aromatic dihydroxy compound is present, the initial amount of component A) obviously is related to conditions (a) to (c).

Moreover, for the skilled person the expression "before step (ii) " is clear. As according to the present invention in step (i) an article, methanol, a catalyst and optionally dimethyl carbonate are brought into contact it is highly preferred that in step (i) the amounts and ratios of those component are already adjusted so that the conditions of step (ii) are met. This is especially the case, if process steps (i) and (ii) cannot be clearly distinguished. Moreover, it is clear to the skilled person that the weight ratios of the components before step (ii) have an influence on the weight ratios of reaction products at the end of step (ii).

"The methanolysis reaction of component A) is theoretically completed to 100 %" means that the entirety of component A) (i. e. 100 %) has been depolymerized into its monomers. This implies that all of the structural units derived from an aromatic dihydroxy compound which are contained in the polycarbonate are converted into the respective aromatic dihydroxy compound. Moreover, this implies that each carbonate group which is present in component A) now is part of dimethyl carbonate (assuming that no side reactions took place). Chain terminators and / or branching points (either intended or due to rearrangement) are ignored when calculating 100 %. This means that it is assumed that component A) only consists of carbonate groups and aromatic dihydroxy compounds from which two hydrogen atoms are removed. As explained above, the point where the methanolysis reaction of component A) is theoretically completed to 100 % does not necessarily mean that in reality the conversion of component A) and/or the yield of the aromatic dihydroxy compound is 100 %. The same holds true for the inventive expression of the "end of step (ii)" at which conditions (a) to (c) are met. For the calculation of conditions (a) to (c) it is assumed that the methanolysis reaction of component A) is theoretically completed to 100 %. This defines the "end of the methanolysis reaction" and thus the "end of process step (ii)". However, this is just to calculate the amounts and ratios of the components at the beginning of step (ii). The real composition at the end of process step (ii) might deviate from the theoretical composition which is obtained when assuming that the methanolysis reaction is completed to 100 %. This indeed preferably is the case, because for the calculation of a 100 % completed methanolysis reaction different assumptions, e. g. omitting the presence of chain terminators or branching points in component A), are used.

Preferably, at the end of step (ii) the conversion of the methanolysis reaction is at least 80 %, more preferably at least 90 %, still preferably at least 95 % and most preferably at least 98 % of the theoretical conversion of the methanolysis reaction of component A) as mentioned above. It is beneficial to have a high "real" conversion in order for the process to be economically and/or ecologically efficient.

The skilled person knows how to calculate the weight ratios according to the conditions (a) to (c) by taking into account the amounts of methanol, component A), component B) and the optionally present dimethyl carbonate present before step (ii). As explained above, some further assumptions with respect to component A), other additives in the article different from A) and B) and /or side reactions are to be made. By way of example, in the following an explanation is given how to calculate the conditions (a) to (c) for an article: $0.5 \leq \frac{{m}_{1} \left(DMC\right)}{m \left(component B\right)} \leq 20$ $0.79 \leq \frac{{m}_{1} \left(MeOH\right)}{m \left(component B\right)} \leq 4$ $0.6 \leq \frac{{m}_{1} \left(MeOH\right) + {m}_{1} \left(DMC\right)}{m \left(component B\right) + m \left(dihydroxy compound\right)} \leq 20$ With ${m}_{1} \left(DMC\right) = \frac{m \left(PC\right)}{M \left(PC\right)} ∗ M \left(DMC\right) + {m}_{0} \left(DMC\right)$, where m₁(DMC) is the theoreticalmass of DMC at the end of step (ii) (assuming 100 % conversion of the PC), and m₀(DMC) is the mass of the DMC in step (i), m(component B) is the mass of component B) added in step (i), m(PC) is the mass of polycarbonate added in step (i), M(PC) is the molecular weight of the repeating unit of PC being the aromatic dihydroxy compound with a carbonate group, without 2H and M(DMC) the molecular weight of DMC being 90.08 g/mol; with ${m}_{1} \left(MeOH\right) = {m}_{0} \left(MeOH\right) - 2 ∗ \left(\frac{m \left(PC\right)}{M \left(PC\right)}∗M\left(MeOH\right)\right)$, where m₁(MeOH) is the theoretical mass of MeOH at the end of step (ii) (assuming 100 % conversion of the PC), and m₀(MeOH) is the mass of the MeOH to step (i); with m(dihydroxy compound) is the theoretical mass of the aromatic dihydroxy compound at the end of step (ii) (assuming 100 % conversion of the PC).

As can be seen from above, in case of doubt for the calculation of conditions (a) to (c) any presence of a chain terminator and/or branching point in component A), the catalyst, any water present and any further components in the article which are different from component A) and B) are not considered. In this context "branching point" preferably refers to any branching agent and/or rearrangement structure.

In case an article comprising end-of-life material or any other article of unknown composition is used in step (i) of the present invention, the composition of the article has to be analyzed with respect to the kind and amount of component A) and component B) prior to process step (i) (cf. above). The skilled person knows suitable methods for such analysis, for example infrared spectroscopy as explained above. The amount of polycarbonate present in an article is the basis for calculating the weight of the aromatic hydroxy compound to be produced by the methanolysis reaction. If the exact amount of component B) cannot be determined, preferably the maximum possible amount thereof is considered. The maximum possible amount of component B) is preferably determined by the weight difference of the article and the amount of polycarbonate present therein, for example if the amount of polycarbonate in the article has been analyzed to be 60 wt.-%, the maximum possible amount of component B) is 40 wt.-%. Using this assumption, it can be guaranteed that component B) is indeed in a liquid state at the end of process step (ii).

In condition (a), the weight ratio of dimethyl carbonate : component B) is in the range of 0.5 : 1 to 20 : 1, preferably in the range of 0.7 : 1 to 10 : 1, more preferably in the range of 0.8 : 1 to 4 : 1.

In condition (b), the weight ratio of methanol : component B) is in the range of 0.79 : 1 to 4 : 1, preferably in the range of 1 : 1 to 3.9 : 1, more preferably in the range of 1.1 : 1 to 3.6 : 1.

In condition (c), the weight ratio of (methanol + dimethyl carbonate) : (component B) + aromatic dihydroxy compound) is in the range of 0.6 : 1 to 20 : 1, preferably in the range of 0.61 : 1 to 5 : 1, more preferably in the range of 0.62 : 1 to 3 : 1.

It is preferred that a combination of the preferred ranges of the conditions (a) to (c) is met.

Preferably, the conditions (a) to (c) of the present invention are:
(a) the weight ratio of dimethyl carbonate : component B) is in the range of of 0.7 : 1 to 10 : 1, more preferably in the range of 0.8 : 1 to 4 : 1,(b) the weight ratio of methanol : component B) is in the range of 1 : 1 to 3.9 : 1, more preferably in the range of 1.1 : 1 to 3.6 : 1 and
(c) the weight ratio of (methanol + dimethyl carbonate) : (component B) + aromatic dihydroxy compound) is in the range of 0.61 : 1 to 5 : 1, more preferably in the range of 0.62 : 1 to 3 : 1.

Surprisingly, it was found that it is beneficial that component B) is in a dissolved and/or emulsified state (i.e. in the liquid phase) at the end of process step (ii). Due to conditions (a) to (c) component B) can be in a liquid phase also during process step (ii) (i.e. after an initial reaction time which is preferably short). Firstly, it was found that the reaction velocity of the methanolysis is faster in case component B) is in the liquid phase during and/or at the end of the methanolysis (when compared to the same reaction where component B) not in the liquid phase). As can be seen from the experiments, the amount of aromatic dihydroxy compound which is readily separated from component B) after step (ii) is high. It is even higher when compared to a process step (ii) in which component B) is not in a liquid phase (e. g. as slurry or as coating at the reactor wall). This already leads to a higher yield of the aromatic dihydroxy compound, because the loss of the aromatic dihydroxy compound which sticks to component B) is smaller. Moreover, the examples surprisingly show that a high amount of aromatic dihydroxy compound which is stick to component B) can be removed. This is especially the case when compared to a process in which component B) is not in the liquid phase during and/or at the end of the methanolysis reaction. In the end this means that the inventive process provides for a high yield of aromatic dihydroxy compound. Preferably, the inventive process provides a yield of the aromatic dihydroxy compound of at least 75 %, more preferably at least 80 %, still preferably of at least 85 %, still preferably of at least 90 %, still preferably of at least 95 % and most preferably of at least 98 %. For the skilled person it is clear that the number of washing steps of component B) contributes to this yield. However, it is not economically and/or ecologically reasonable to have a high number of washing steps. At the same time the purity of component B) which is recovered is very high. This means that the effort for any work up of component B) in order to recycle this component is low.

According to the present invention it was found that due to the reaction of the methanolysis the solution conditions of process step (ii) continuously change. With the aim to bring and/or keep component B) in a liquid state, this needs to be considered. The terms "dissolve" and/or "in solution" and/or "in a liquid state" preferably mean that, when filtering the liquid in which component B) is present, no solid can be separated off using customary filter methods and/or that the liquid phase remains uniform over time without phase separation. Moreover, it is preferred that the term "in a liquid state" refers to room temperature. Dimethyl carbonate is a good solvent for component B), whereas methanol is an anti-solvent. This also means that depending on the chemical nature of component B) dimethyl carbonate can form an emulsion with component B), whereas methanol does not form an emulsion with component B). The term "anti-solvent" is understood by the skilled person. It preferably refers to a liquid which reduces the solubility product of component B) in a given (highly concentrated) solution of component B). This preferably means that it is capable of precipitating component B) from a (highly concentrated) solution of component B). The generated aromatic dihydroxy compound is a good solvent for component B), too. During the methanolysis reaction the amounts and ratios of dimethyl carbonate, methanol and the aromatic dihydroxy compound change. Methanol is consumed, whereas dimethyl carbonate and the aromatic dihydroxy compound is generated. Accordingly, the conditions constantly vary. However, when using the conditions (a) to (c) according to the present invention, the presence of component B) in a liquid state at least at the end of process step (ii) is guaranteed. The amount of component B) in the initial article is decisive whether dimethyl carbonate needs to be added from or whether the generated dimethyl carbonate is sufficient. In the case where additional dimethyl carbonate is needed to guarantee that component B) is in the liquid state at the end of the methanolysis reaction, it can be added at the very beginning of step (ii) and/or during process step (ii). Moreover, conditions (a) to (c) guarantee that the process is still economically and ecologically advantageous. A high amount of methanol could lower the viscosity of the mixture as obtained after inventive step (ii). However, it could lead to precipitation of component B) and will need to be removed afterwards from the aromatic dihydroxy compound. Therefore, in order to keep the process economic and ecological, the amount of methanol should be as low as possible (in the boundaries of the methanolysis reaction).

Moreover, it was found according to the present invention that when using conditions (a) to (c) the resulting viscosity after performing step (ii) is low and thus, very well manageable. Preferably, the viscosity after performing step (ii) is in the range of not more than 1000 mPa*s, more preferably of not more than 500 mPa*s, still more preferably of not more than 300 mPa*s, still more preferably of not more than 100 mPa*s and most preferably of not more than 50 mPa*s. Preferably, these viscosity numbers refer to the temperatures used in step (ii). Methods how to determine the viscosity are known to the skilled person. It is apparent that the viscosities are greater than 0 mPa*s and that the lower the viscosity, the easier the workup after step (ii) will be. These indicated viscosities are manageable and at the same time the amount of methanol is not too high so that the process is still economically and ecologically advantageous.

According to the present invention it was found that the amount of methanol and dimethyl carbonate have more influence on the dissolution of component B) than the aromatic dihydroxy compound.

In order to adjust the ratio of dimethyl carbonate : component B) and the ratio of (methanol + dimethyl carbonate) : (component B) + aromatic dihydroxy compound) according to the conditions (a) and (c) of the present invention, dimethyl carbonate (DMC) may be added to the reaction mixture of step (i) in the required amount. It is preferable to keep the amount of dimethyl carbonate added to the reaction mixture of step (i) at a minimum. This increases the production efficiency. Additional amounts of dimethyl carbonate lead to an increased volume of the reaction mixture and thus the amount of energy required for heating and mixing, as well as to increased efforts necessary for subsequent workup, e.g. separation of solvents, separation of component B) and side products from the desired products. Furthermore, it is unfavorable to add too large amounts of dimethyl carbonate to the reaction mixture of step (i) as this means that the amount of dimethyl carbonate generated during the methanolysis reaction of step (ii) has to be low. In this case the content of component A) in the article with respect to the content of component B), both present in the reaction mixture of step (i), is low. Thus, the amount of aromatic dihydroxy compound to be recovered is also low. This has a negative impact on the absolute amount of the aromatic dihydroxy compound to be obtained based on the mass of the whole article making it less economical attractive. It is therefore preferable to add 0 to less than 1.8 mol of dimethyl carbonate relative to 1 mol of aromatic dihydroxy compound.

Alternatively, the ratios of conditions (a) and (c) of the present invention may be adjusted by adjusting the ratio of component A) : component B) in the reaction mixture of step (i). As component A) is depolymerized during the methanolysis reaction in step (ii), dimethyl carbonate is generated and thus contributes to the ratios of dimethyl carbonate : component B) and (methanol + dimethyl carbonate) : (component B) + aromatic dihydroxy compound), respectively. The skilled person can calculate the amount of dimethyl carbonate which is produced under the assumption that the methanolysis reaction of component A) is completed to 100 %. The ratio of component A) : component B) can for example be adjusted by adding component A) to the reaction mixture of step (i) or by mixing articles with different component A) : component B) ratios. By this the total amount of dimethyl carbonate which is needed can also be influenced and conditions (a) to (c) can be accordingly adjusted. It is preferred to adjust the ratio of component A) : component B) by increasing the amount of component A) rather than to increase the amount of dimethyl carbonate in order to fulfil conditions (a) to (c).

Furthermore, it is preferred that the process according to the present invention is characterized in that the amounts of methanol, component A), component B) and the optionally present dimethyl carbonate before step (ii) are chosen so that the following conditions (a) to (d) are met at the end of step (ii), wherein the conditions (a) to (d) represent weight ratios which are obtained using the assumption that the methanolysis reaction of component A) is theoretically completed to 100 %:
wherein conditions (a) to (c) are as described above and
(d) the weight ratio of dimethyl carbonate : methanol : component B) is in the range of 0.015 to 0.5, more preferably in the range of 0.02 to 0.38 and most preferably in the range of 0.02 to 0.35.

This weight ratio is understood as being the quotient of the mass of dimethyl carbonate, the mass of methanol and the mass of component B) and the respective quotient needs to be in the indicated range.

According to the present invention, this additionally preferred condition (d) specifically leads to an economically and ecologically advantageous process.

It is preferred that the amount of component A) in the article is at least 25 wt.-%, more preferably at least 30 wt.-% and most preferably 36 wt.-%. A high amount of component A) in the article increases the overall yield and thus production efficiency of the recovery of the hydroxy compound. More preferably in order to get an economically attractive process, the amount of component A) in the article is at least 40 wt.-%, more preferably at least 50 wt.-% and most preferably 60 wt.-%. These amounts of component A) preferably relate to the total content of component A) in case a mixture of articles is used.

### Further process steps

It is preferred that the process according to the present invention further comprises the steps of
(iii) precipitating component B) from the product as obtained after performing step (ii) to obtain a suspension comprising a solid fraction comprising the rubber-modified graft polymer and optionally the at least one vinyl(co)polymer and a liquid fraction comprising the aromatic dihydroxy compound and
(iv) separating the solid fraction and the liquid fraction of the suspension of step (iii).

The precipitation of component B) can be achieved by any means known to the skilled person. Preferably, the precipitation of component B) is achieved by lowering the solubility of component B) in the mixture as obtained after performing step (ii), e.g. by lowering the temperature and/or changing the solubility conditions. This can be done either by addition of an anti-solvent or by removal of a solvent. As also defined before "anti-solvent" preferably means a solvent in which a compound is less soluble. If an anti-solvent is used for the precipitation of component B), the anti-solvent preferably is a substance which is present at the end of step (ii) of the inventive process. Most preferably, the anti-solvent is methanol and/or water. Preferably, the precipitation of component B) is achieved by the addition of methanol. This is advantageous, because methanol is already present in the system. This means that the further work-up is advantageous, because no further components need to be removed. It is also advantageous for recovering the methanol. For example, the methanol could be recycled and used in process step (i). Using methanol as anti-solvent in process step (iii) does not add any further components which might have a negative impact on the recyclability of the methanol. However, as indicated before, when recycling methanol the amount some side products and /or water could increase in process step (i).

According to the present invention it was found that due to the inventive process a precipitation of component B) is possible. This results in a processable suspension. Preferably, this means that the suspension can be pumped and/or removed from the reactor. In comparison to this if component B) is not in the liquid phase at the end of process step (ii) it sticks to the reactor wall and is difficult to be removed from the reactor. Moreover, this filterability leads to less incorporation of any educts, products and/or catalysts in the resulting rubber-modified graft polymer and optionally at least one vinyl(co)polymer. Moreover, those educts, products and/or catalyst can be easily removed by washing.

The separation of the solid fraction and the liquid fraction of the suspension (process step (iv)) can be done by any solid/liquid separation process known to the skilled person, e.g. filtration, sedimentation, decantation and/or centrifugation. Preferably, the solid fraction and the liquid fraction are separated using filtration. It was found that when using the process of the present invention precipitated component B) can be easily filtered off and/or washed. This is due to the fact that component B) forms a fine, powdery residue in form of a suspension of low viscosity. In this context the term "low viscosity" preferably refers to a viscosity of < 20 mPas. It is known to the skilled person that filtration of mixtures with viscosities of > 20 mPas is technically not reasonable. In contrast to this, if component B) is not in the liquid phase after process step (ii) (e. g. comparative examples), it forms a sticky, phase-separated mass of high viscosity (> 20 mPas). Due its high viscosity this mass is not filterable, it cannot be washed with any washing agent and thus, the desired products which might be still present in this mass cannot be removed (e. g. the aromatic dihydroxy compound cannot be washed off component B)). Optionally, the solid fraction can be washed after the separation step in order to remove any remaining anti-solvent or other compounds present at the end of step (ii) of the inventive process which are not component (B), e.g. aromatic dihydroxy compound, methanol, catalyst and dimethyl carbonate, water, residual oligocarbonates. Preferably, the solid fraction is washed after the separation step. This additional step of washing can be used in order to increase the purity of the resulting inventive composition because by washing, remaining aromatic dihydroxy compound and/ or catalyst can be removed. For the washing for example methanol and/or water can be used. The amount of methanol and/or water in the resulting inventive composition can be reduced by drying the composition.

Particular preferably, the process of the present invention is a process wherein inventive composition is recovered and which additionally comprises the steps of isolating the the rubber-modified graft polymer and optionally the at least one vinyl(co)polymer. Most preferably, in this case, the inventive process comprises inventive process steps (iii) and (iv) and a step of isolating the rubber-modified graft polymer and at least one vinyl(co)polymer.

It was found that when using the inventive process the recyclability of the rubber-modified graft polymer and optionally at least one vinyl(co)polymer is very good. As explained above, it was found that component B) can be easily separated from any other component that are soluble in the washing liquid present after process step (ii). It has a good quality. It was found that it has a low residual content of aromatic dihydroxy compound. Moreover, it has a low residual content of potentially other low molecular, organic compounds beside the aromatic dihydroxy compound (such as for example chain terminator and / or potential additives of the article such as component C)).

In a further aspect of the present invention, the inventive composition further comprising at least one further polymeric component distinct from B.1 and B.2 and/or a polymer additive.

Preferably, the polymer additive and further polymeric component is preferably any additive or further polymeric component as described above with respect to component C). More preferably, the "further polymeric component" is a thermoplastic polymer, most preferably polycarbonate.

Moreover, in a further aspect of the present invention a thermoplastic molding compound is provided which is obtained from the inventive composition. Finally, the present invention provides a molded article obtained from the inventive thermoplastic molding compound. The molded articles containing the inventive thermoplastic molding compound can be manufactured for example by injection molding, extrusion, and blow-molding processes. Another form of processing is the production of molded articles by thermoforming from previously produced plates or films.

According to the present invention the term "comprising" refers to compositions or process steps which at least contain the indicated features. Moreover, "comprising" preferably revers to "consisting essentially of". Most preferably, the term "comprising" means "consisting of.

Description of figures:

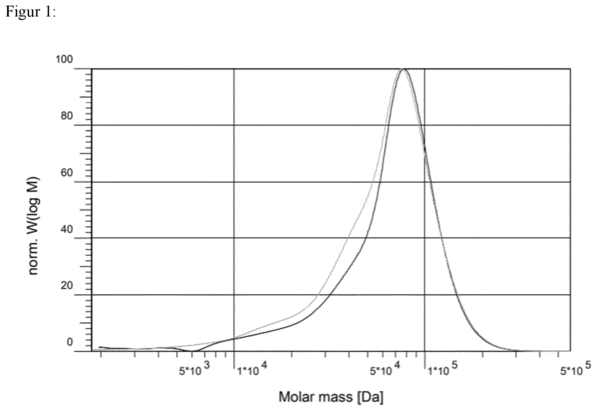

Figure 1: Figure 1 shows the GPC spectrum of virgin ABS (light grey) and ABS as obtained by inventive example 1 (grey). A clear difference can be seen at a molar mass around 30 000 Da and 50 000 Da. While the virgin ABS comprises a "shoulder" which can be attributed to oligomeric SAN, in the recycled ABS this shoulder is smaller.

### Examples

### Analytics:

### Nuclear Magnetic Resonance Spectroscopy (1H NMR):

The NMR measurements were performed on a Spinsolve 80 Ultra device from magritek at 80 MHz at 300 K. The spectra were normalized to the corresponding resonances of the solvent: 1H: δ = 2.50 ppm (DMSO-d6) or δ = 3.31 ppm (MeOH-d4). The chemical shift was given in ppm. For quantification, a known amount of pyrazine (1H: δ = 8.5 ppm (s)) was added as an internal standard. The NMR spectra were evaluated with the MestReNova software.

### High-Performance Liquid Chromatography (HPLC):

The quantification of Bisphenol A (BPA), 1,3-butadiene, Acrylnitrile, 4-Vinyl-1-cyclohexane, Ethylbenzol, Chlorobenzene and Styrene in Acrylonitrile Butadiene Styrene (ABS) was performed using an Agilent Technologies 1260 Infinity II HPLC system. The system included a G7112B pump, G7129A sampler, G7116A thermostat, and G7117A UV detector. A Restek UHPLC PreColumn filter (0.2 µm) was used as a prefilter. The chromatographic separation was achieved on an Agilent ZORBAX Eclipse Plus C18 column (150 mm × 4.6 mm, 5 µm particle size, 95 Å pore size) at 35 °C. The pressure limit was set to ≥ 400 bar. The mobile phases consisted of ultrapure water (Solvent A) and 100 % methanol (Solvent B). The injection volume was 5 µL, and the sampler temperature was maintained at 35 °C. Detection was performed using a diode array detector (DAD) with signals at 254 nm, 278 nm, and 240 nm. The spectrum was recorded from 190 to 400 nm with a data collection rate of 2.5 Hz.

Sample preparation involved weighing approximately 50 mg of ABS into a 20 mL screw-cap vial, adding 10 mL of acetonitrile, and sonicating for 10 minutes. The solution was then diluted with water to precipitate the ABS. The diluted solutions were filtered with 0.2 µm PTFE filters before injection. Quantification was performed using an external calibration with seven BPA standards (5.3 to 33.6 ppm). The calibration standards were prepared in the same solvent composition as the sample extracts.

### Gas Chromatography (GC):

The analysis of isolated Bisphenol-A (BPA), was conducted using an Agilent 6890 gas chromatograph equipped with a flame ionization detector (FID), an automatic injector, and AGILENT Chemstation software (version A.06.03). The chromatographic separation was achieved using a WCOT quartz glass column (Chrompack:nr7749, now Agilent) with CP-Sil 5CB as the stationary phase, an internal diameter of 0.32 mm, a film thickness of 0.4 µm, and a length of 50 meters. For the analysis of BPA in its solid form, 10 mg (±1 mg) of the dried product was placed into a GC vial, followed by the addition of 150 µL of silylation reagent (N-Methyl-N-trimethylsilyltrifluoracetamid (MSTFA)). The mixture was heated on the GC detector's heating block for 10 minutes until fully dissolved, then 250 µL of ethyl acetate was added. The analysis was conducted using hydrogen as carrier gas, with a column flow rate of 4.5 mL/min and a total flow rate of 50 mL/min. The detector gases included air at 400 mL/min, hydrogen at 40 mL/min, and nitrogen at 45 mL/min. The AGILENT ChemStation software was used to automatically calculate the content in percentage, excluding the peaks of the silylation reagent. Any peaks not listed in the calibration table were disregarded, and the chromatogram was visually inspected for foreign components. Calibration and verification were performed according to the specified frequency, using reference samples.

### FT-IR spectroscopy:

The IR spectra were recorded with an FT-IR spectrometer Alpha II from Bruker at room temperature. The ATR (diamond) measurement principle was used. The spectrum was recorded from 400 cm⁻¹ to 4000 cm⁻¹. The analysis of the spectrum was enabled by the OPUS 7,5 and Origin software. The absorption was given in cm-1. The band at 1512 cm⁻¹ for BPA was considered, which represents the HCH bending vibration.

### Sodium analysis by ICP-OES:

Sodium content analysis was performed by an external accredited laboratory (Currenta GmbH & Co. OHG, Leverkusen, Germany) using inductively coupled plasma optical emission spectrometry (ICP-OES) according to their validated method (reference number: 2011-0479301-94D). The analysis was conducted following standardized procedures in compliance with DIN EN ISO/IEC 17025 requirements for testing laboratories.

### Gel Permeation Chromatography (GPC)

Molecular weight distributions were determined using an Agilent 1260 Infinity SECcurity GPC system (Agilent Technologies). The separation was achieved using a combination of two PSS SDV columns with 100 Å pore size and two PSS SDV columns with 1000 Å pore size (all columns: 5 µm particle size). Tetrahydrofuran (THF) containing toluene as internal standard served as the mobile phase at a flow rate of 1.0 mL/min and 40 °C. Samples (10-20 mg) were dissolved in THF and filtered through a 200 nm PTFE membrane prior to injection. The injection volume was 100 µL. Calibration was performed using polystyrene standards in the molecular weight range of 266 - 66,000 g/mol. The polydispersity index (PDI) was calculated as the ratio of weight-average molecular weight to number-average molecular weight (M̅w/M̅n) considering the molecular weight range from 2 × 10³ to 3 × 10⁵ g/mol using PSS WinGPC UniChrom software for data analysis.

### Dynamic Mechanical Analysis

Glass transition temperatures Tg for recovered ABS and blends with polycarbonate and the recovered ABS were determined by using dynamic-mechanical analysis (DMA) in non-resonant torsion mode (shear modulus G*(T)) according to standard DIN EN ISO 6721-7 at 1Hz frequency with a heating rate of 3K/min in a temperature range from -150°C to softening of the material as temperatures related to the peak maximum in the loss modulus (G") curve.

### Particle Size Analysis

Particle size distribution of non-spherical ABS particles was determined using dynamic light scattering (DLS). Sample preparation was performed using an automated wet dispersion unit (Hydro MV, Malvern Panalytical). The samples were dispersed in water following a standardized protocol: initial dispersion at 2000 rpm combined with ultrasonication (50% power output) for 120 seconds, followed by continuous stirring at 2000 rpm for an additional 12 minutes. Measurements were conducted at room temperature using a Mastersizer 3000 laser diffraction analyzer (Malvern Panalytical) with the manufacturer's software (21 CFR Part 11). The Mie scattering theory was applied for particle size calculations. Results represent the average of six independent measurements at an obscuration level below 10%.

### Materials:

Sodium hydroxide (50 wt.% NaOH in H₂O) and methanol (98 %) were purchased from Fischer Scientifics. Dimethyl carbonate (99 %) and toluene (≥99.5) were purchased from Sigma Aldrich.

PC composition 1:
A) 69.7 wt.% of polycarbonate based on bisphenol A
B) 29.9 wt.% of ABS made by bulk polymerization having an acrylonitrile:butadiene:styrene ratio of 23:10:67. The weight average molecular weight of the free styrene acrylonitrile copolymer is 160,000 g/mol (measured by gel permeation chromatography (GPC) in tetrahydrofuran against a polystyrene standard). A sum of residual monomer content including 1,3-butadiene, Acrylnitrile, 4-Vinyl-1-cyclohexane, Ethylbenzol, Chlorobenzene and Styrene of 400 ppm was analyzed by HPLC.
C) 0.4 wt.% of additives

PC composition 2:
A) 49.05 wt.% of polycarbonate based on bisphenol A
B) 49.05 wt.% of ABS made by bulk polymerization having an acrylonitrile:butadiene:styrene ratio of 23:10:67. The weight average molecular weight of the free styrene acrylonitrile copolymer is 160,000 g/mol (measured by gel permeation chromatography (GPC) in tetrahydrofuran against a polystyrene standard)
C) 1.9 wt.% of additives

PC composition 3:
A) 42.75 wt.% of polycarbonate based on bisphenol A
B) 42.75 wt.% of ABS made by bulk polymerization having an acrylonitrile:butadiene:styrene ratio of 23:10:67. The weight average molecular weight of the free styrene acrylonitrile copolymer is 160,000 g/mol (measured by gel permeation chromatography (GPC) in tetrahydrofuran against a polystyrene standard)
C) 14.5 wt.% of additives

PC composition 4:
A) 44.3 wt.% of polycarbonate based on bisphenol A
B) 44.3 wt.% of ABS made by bulk polymerization having an acrylonitrile:butadiene:styrene ratio of 23:10:67. The weight average molecular weight of the free styrene acrylonitrile copolymer is 160,000 g/mol (measured by gel permeation chromatography (GPC) in tetrahydrofuran against a polystyrene standard
C) 11.4 wt.% of additives

PC composition 5:
A) 36.8 wt.% of polycarbonate based on bisphenol A
B) 36.8 wt.% of ABS made by bulk polymerization having an acrylonitrile:butadiene:styrene ratio of 23:10:67 and 24.5 wt.% of poly(methyl methacrylate) with 1 wt.% methyl acrylate content (Plexiglas^{®} 8H, Röhm). The weight average molecular weight of the free styrene acrylonitrile copolymer is 160,000 g/mol (measured by gel permeation chromatography (GPC) in tetrahydrofuran against a polystyrene standard. The weight average molecular weight of the poly(methyl methacrylate) is 147,000 g/mol (measured by gel permeation chromatography (GPC) in tetrahydrofuran under PMMA calibration).
C) 1.9 wt.% of additives

PC composition 6:
This composition consists of recovered end-of-life material. It was analyzed and its composition was determined as follows: potassium bromide pellets were prepared from the ground original, and infrared spectra was recorded in transmitted light. Using a calibration of known polycarbonate contents, the PC content could be determined. Furthermore, a defined amount of the composition was depolymerized by means of sodium methoxide under reflux.

The residue was separated from the solution, washed and dried. Using IR, the residue was found to be essentially PMMA due to the presence of the ester stretching frequency. The amount of PMMA was quantified by gravimetry and set as component B). The resulting content of the single components was found to be approximately as follows:
A) 36 wt.% of polycarbonate based on bisphenol A
B) max. 64 wt.% of poly(methyl acrylate)/poly(methyl methacrylate)

For the depolymerization of this material, the maximum possible amount of component B) was considered. This means that everything that depolymerized was assumed to be component A) and the whole rest of the sample which was not component A) was assumed to be component B).

PC composition 7:
This composition consists of recovered end-of-life material. It was analyzed and its composition was determined as follows: potassium bromide pellets were prepared from the ground original, and infrared spectra were recorded in transmitted light. Using a calibration of known polycarbonate contents, the PC content could be determined. The rest of the IR spectrum gave indication to the presence of ABS as confirmed by the presence of the C-N stretching vibration at v = 2240 cm⁻¹. The content of the single components was found to be approximately as follows:
A) 75 wt.% of polycarbonate based on bisphenol A
B) max. 25 wt.% of ABS

For the depolymerization of this material, the maximum possible amount of component B) was considered. This means that the whole rest of the sample which was not component A) was assumed to be component B).

PC composition 8:
A) 81.95 wt.% of polycarbonate based on bisphenol A
B) 8.65 wt.% of ABS made by bulk polymerization having an acrylonitrile:butadiene:styrene ratio of 23:10:67. The weight average molecular weight of the free styrene acrylonitrile copolymer is 160,000 g/mol (measured by gel permeation chromatography (GPC) in tetrahydrofuran against a polystyrene standard)
C) 9.4 wt.% of additives

PC composition 9:
A) 60.19 wt.% of polycarbonate based on bisphenol A
B) 38.51 wt% of ABS comprising ABS made by bulk polymerization having an acrylonitrile: butadiene: styrene ratio of 23:10:67. The weight average molecular weight of the free styrene acrylonitrile copolymer is 160,000 g/mol (measured by gel permeation chromatography (GPC) in tetrahydrofuran against a polystyrene standard)
C) 1.3 wt.% of additives

PC composition 10:
A) 81.95 wt.% of polycarbonate based on bisphenol A
B) 8.65 wt.% of ABS made by bulk polymerization having an acrylonitrile:butadiene:styrene ratio of 23:10:67. The weight average molecular weight of the free styrene acrylonitrile copolymer is 160,000 g/mol (measured by gel permeation chromatography (GPC) in tetrahydrofuran against a polystyrene standard)
C) 9.4 wt.% of additives

PC composition 11:
PC composition 11 was prepared analogously to PC composition 1 but instead of virgin ABS the recycled ABS obtained by inventive example 1 was used. The blend was prepared using a laboratory-scale co-rotating twin-screw mini extruder P11 (Thermo Fisher Scientific, Karlsruhe, Germany) equipped with standard screws. The processing was conducted at a barrel temperature of 260 °C with a throughput of 0.6 kg/h and a screw speed of 200 rpm. The melt temperature at the die head was measured to be 245 °C. During extrusion, the melt was devolatilized under vacuum conditions of 10 mbar absolute pressure. The process was operated at a torque range of 46-53% and melt pressures between 23-30 bar.
A) 70 wt.% of polycarbonate based on bisphenol A
B) 30 wt.% of ABS obtained by inventive example 1 with following residual monomer contents (analyzed by HPLC):

| **Residual momomers in PC composition 11** | **Amounts [ppm]** |
|---|---|
| 1,3- Butadiene | <0,20 |
| Acrylnitrile | <5,0 |
| 4-Vinyl-1-cyclohexane | <5,0 |
| Ethylbenzene | <5,0 |
| Chlorbenzene | <5,0 |
| Styrene | <5,0 |

PC composition 12:
PC composition 12 was prepared analogously to PC composition 1 but instead of virgin ABS the recycled ABS obtained by comparative example 7 (CE-7) was used. The blend was prepared using a laboratory-scale co-rotating twin-screw mini extruder P11 (Thermo Fisher Scientific, Karlsruhe, Germany) equipped with standard screws. The processing was conducted at a barrel temperature of 260 °C with a throughput of 0.6 kg/h and a screw speed of 200 rpm. The melt temperature at the die head was measured to be 245 °C. During extrusion, the melt was devolatilized under vacuum conditions of 10 mbar absolute pressure. The process was operated at a torque range of 46-53% and melt pressures between 23-30 bar.
A) 70 wt.% of polycarbonate based on bisphenol A
B) 30 wt.% of ABS obtained by comparative example 7

### Depolymerization:

Inventive Example 1: In a 1 L reactor, 280.80 g of ground PC composition 1, 259.20 g of methanol (MeOH) and 1.20 g sodium hydroxide (NaOH 50 %) in water as a catalyst were added. The reaction mixture was stirred at 400 rpm and heated to 110 °C for 240 minutes or until a constant bisphenol A (BPA) concentration was reached. The BPA concentration was monitored by online-IR. The reaction solution was then cooled to ≤ 25 °C.

For this example conditions (a) to (c) were calculated as follows:$m \left(component A\right) = m \left(composition\right)$ $m \left(component A\right) = m \left(composition 1\right) ∗ 0.697 = 195.71 g$ $M \left(component A\right) = 254 g / mol$ $M \left(DMC\right) = 90.08 g / mol$ ${m}_{0} \left(DMC\right) = 0 g$ $m \left(component B\right) = m \left(composistion 1\right) ∗ 0.299 = 83.96 g$ $\begin{matrix}{m}_{1} \left(MeOH\right) = {m}_{0} \left(MeOH\right) - 2 ∗ \left(\frac{m \left(component A\right)}{M \left(component A\right)}∗M\left(MeOH\right)\right) \\ = 259.20 g - 2 ∗ \frac{195.71 \frac{g}{mol}}{254 \frac{g}{mol}} ∗ 32.04 \frac{g}{mol} = 209.83 g\end{matrix}$ $\begin{matrix}m \left(BPA\right) = m \left(component A\right) ∗ \frac{M \left(BPA\right)}{M \left(component A\right)} = 195.71 g \frac{228 \frac{g}{mol}}{254 \frac{g}{mol}} \\ = 175.68 g\end{matrix}$

Calculation of (a) using equation $\left(a\right) = \frac{{m}_{1} \left(DMC\right)}{m \left(component B\right)} = \frac{\frac{m \left(component A\right)}{M \left(component A\right)} ∗ M \left(DMC\right) + {m}_{0} \left(DMC\right)}{m \left(component B\right)} = \frac{\frac{195 , 71 g}{254 g / mol} ∗ 90.08 g / mol}{83.96 g} = 0.83$

Calculation of (b) using equation $\left(b\right) = \frac{{m}_{1} \left(MeOH\right)}{m \left(component B\right)} = \frac{209.83 g}{83.95 g} = 2.50$

Calculation of (c) using equation: $\left(c\right) = \frac{{m}_{1} \left(MeOH\right) + {m}_{1} \left(DMC\right)}{m \left(component B\right) + m \left(BPA\right)} = \frac{209.83 g + 69.41 g}{83.96 g + 175.68 g} = 1.07$

Table 1 shows the different polycarbonate compositions which were used according to the above given depolymerization scheme. Comparable reaction setups as given for inventive example 1 above were used. If dimethyl carbonate was actively added it was added together with PC, MeOH and the catalyst to the reactor. For all examples complete depolymerization of PC to BPA was ensured via online IR.

### Precipitation & Filtration:

The solution obtained from the decomposition of PC composition 1 of inventive example 1 was added to a stirred solution of methanol to give a fine non-sticky suspension. The resulting suspension was stirred for 3 minutes and then filtered through a glass frit. The precipitate was washed two times with methanol (V(filter cake)/V(MeOH) = 1/1). The ABS was resuspended in MeOH (V(filter cake)/V(MeOH) = 5/1) and stirred for 10 minutes. The resulting suspension was filtered and the residue was washed two times with MeOH (V(filter cake)/V(MeOH) = 1/1). The resuspension wash was repeated once more. The resulting residue was dried at 100°C for 2 h in vacuo to yield ABS as powder.

### Comparative Example 7 (CE-7)

Depolymerization:
In a 1 L reactor, 180 g of ground PC composition 1, 217 g of methanol (MeOH), 217 g water and 167 g sodium hydroxide (NaOH 50 %) in water as a catalyst were added. The reaction mixture was stirred at 400 rpm and heated to 77 °C. After 240 minutes slurry was obtained containing solid particles in a liquid solution.

### Filtration:

The suspension was filtered over a paper prepleated filter (type Whatman 595 ½) with pore size 4 - 7 mm leaving the ABS solid particles on the filter paper. The solid ABS particles were washed with 1200 g of methanol and 5 times with 1600 g of demineralized water. The ABS particles were dried overnight in an oven at 70°C.

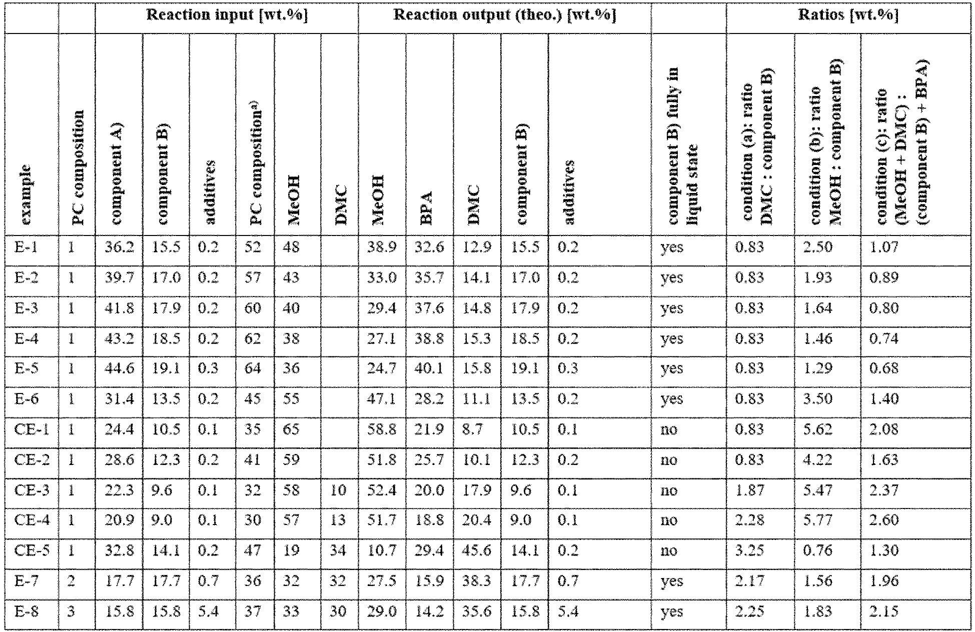

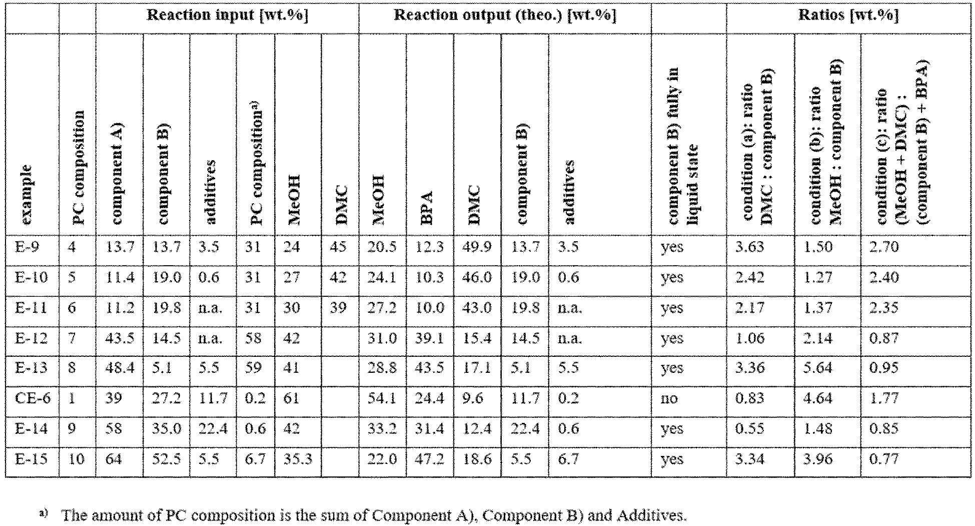

As can be seen from table 1, only when using the inventively defined conditions (a) to (c) component B) is in the liquid state after performing step (ii).

### Washing experiments:

### Inventive experiment E-1:

The reaction product of inventive example E-1 after depolymerization (inventive step (ii)) was added to a stirred solution of MeOH resulting in the formation of a suspension. The suspension was filtered and the BPA content of the solid was analyzed by HPLC to quantify the theoretical loss of BPA yield without washing (cf. Table 2, indicated there as "without washing"). The solid ABS residue was washed with MeOH (v/v of MeOH/wet ABS residue = 1/1) dried at 100°C in the vacuum oven for 2 hours and the BPA content in the ABS residue was analyzed by HPLC to quantify the theoretical loss of BPA yield after 1^{st} washing step (cf. Table 2 1^{st} washing step). The resulting ABS residue was mixed with MeOH (v/v of MeOH/wet ABS residue = 5/1) and was stirred for 10 minutes and filtered. The BPA content of the ABS residue (dried) was analyzed by HPLC to quantify the theoretical loss of BPA yield after 2^{nd} washing (cf. Table 2, 2^{nd} washing step).

For this example loss of BPA yield was calculated as follows:Loss of BPA yield = m(BPA in ABS)/ m(BPA total) $Loss of BPA yield \left[wt%\right] = \frac{m \left(BPA in ABS\right)}{m \left(BPA total\right)}$

Where m(BPA in ABS) is the mass of BPA in the ABS residue after filtration which was determined by HPLC-MS and m(BPA total) is the theoretical mass of BPA obtained after complete conversion of component A to BPA.

### Comparative experiment CE-6:

After the depolymerization reaction (inventive step (ii)) as described above, the resulting reaction solution at 25 °C remained biphasic containing a clear and low viscous upper layer (MeOH phase) and a white, high viscous and non-flowable bottom layer (ABS phase). The BPA content of the bottom layer was analyzed by ¹H NMR spectroscopy to quantify the theoretical loss of BPA yield without washing (cf. Table 2, without washing).

The upper layer (MeOH phase) of the biphasic reaction mixture was removed by decantation. MeOH (v/v of MeOH/bottom layer = 5/1) was added to the ABS-rich phase and the mixture was mixed using a orbital shaker for 10 minutes. The upper layer (MeOH phase) was separated by decantation and the BPA content of the bottom layer was analyzed by ¹H NMR spectroscopy to the quantify the theoretical loss of BPA yield after the 1^{st} washing step. (cf. Table 2, 1^{st} washing step). The washing step was repeated followed by analysis of the BPA content of the bottom layer by ¹H NMR spectroscopy to quantify the theoretical loss of BPA yield after the 2^{nd} washing step. (cf. Table 2, 2^{nd} washing step).

Each time (in the inventive and the comparative example) the theoretical loss of BPA yield was calculated as follows:
For this example loss of BPA yield was calculated as follows: $Loss of BPA yield \left[wt%\right] = \frac{m \left(BPA in ABS\right)}{m \left(BPA total\right)}$

Where m(BPA in ABS) is the mass of BPA in the ABS rich phase after phase separation which was determined by ¹H NMR spectroscopy and m(BPA total) is the theoretical mass of BPA obtained after complete conversion of component A to BPA.

**Table 2**

| | Loss of BPA yield [%] | | |
|---|---|---|---|
| | Without washing | 1^{st} washing step | 2^{nd} washing step |
| **E-1** | 14.3 | 3.8 | 0.9 |
| **CE-6** | 38 | 36 | 23 |

As can be seen from Table 2 the inventive process provides for a higher BPA yield or a lower loss in BPA yield when performing the same numbers of washing steps and compared to a process in which the ABS was not in the liquid form to result a homogeneous solution and/or stable emulsion. As can be seen already the initial loss of BPA yield is much lower for the inventive example when compared to the comparative example. Moreover, the BPA can be removed more easily from the ABS which can be especially seen after the first washing step. In the inventive example the loss of BPA yield is reduced about 10 %, whereas for the comparative example this reduction is only 2 %.

Finally, the recycled ABS as obtained by inventive example 1 and the ABS as obtained by comparative example 7 were compounded with polycarbonate (description cf. above). The results are summarized in Table 3.

**Table 3:**

| | **Virgin ABS** | **ABS obtained by inventive example 1** | **ABS obtained by comparative example 7** |
|---|---|---|---|
| BPA content [ppm] | - | 3000 | ~5000 |
| Na content [ppm] | 2-3 | 68 | ~1500 |
| ABS monomers [ppm] | ~400 | < 5 | n.d. |
| T_{g}-Polybutadien | -91 | -94 | -91 |
| T_{g}-SAN | 108 | 111 | 109 |

| **PC : ABS = 70 : 30** | **PC composition 1** | **PC composition 11** | **PC-composition 12** |
|---|---|---|---|
| BPA content [ppm] | 68 | 1074 | n.d. |
| T_{g}- PC [°C] | 146 | 149 | 129 |

The ABS used for PC composition 11 exhibited a glass transition temperature (Tg) of -94°C for the polybutadiene component and a Tg of 111°C attributed to the SAN component in ABS.

These values were in close agreement with the virgin material used for the preparation of PC component 1, which had Tg values of -91°C for the polybutadiene part and -108°C for the SAN part in ABS. The comparable values confirmed that the ABS structure has not been compromised during the methanolysis process. The slightly higher Tg values for the ABS used for PC component 11 resulted from the successful removal of oligomeric SAN through methanolysis.

Gel permeation chromatography (GPC) analysis further showed the removal of oligomeric SAN, as indicated by the lower polydispersity index (PDI) of 1.49 compared to virgin ABS of 1.74 (cf. figure 1). PC component 11 showed a comparable but slightly higher Tg of 149°C for the PC component, compared to the comparative PC composition 1, which showed a Tg of 146°C. This indicates that the PC containing the inventive composition has not been degraded during compounding, and the slightly higher Tg value again confirmed the removal of SAN oligomers.

## Claims

1. A composition comprising a rubber-modified graft polymer B.1 and optionally a rubber-free vinyl(co)polymer B.2, comprising more than 0 ppm methanol, more than 0 ppm of at least one aromatic dihydroxy compound and more than 0 ppm and less than 1000 ppm sodium, wherein the ppm are based on the amount of the sum of B.1 and B.2.

2. The composition of claim 1, wherein the amount of methanol is more than 0.1 ppm and equal to or less than 10 000 ppm.

3. The composition of claim 1 or 2, wherein the amount of the at least one aromatic dihydroxy compound is more than 1 ppm and equal to or less than 10 000 ppm.

4. The composition of any one of claims 1 to 3, which additionally comprises more than 0 ppm dimethylcarbonate.

5. The composition of any one of claims 1 to 5, which additionally comprises less than 400 ppm vinyl monomer.

6. The composition of any one of claims 1 to 5, wherein the at least one aromatic dihydroxy compound comprises a bisphenol, preferably bisphenol A.

7. The composition of any one of claims 1 to 6, wherein the rubber-modified polymer B.1 comprises
B.1.1 5 to 95% by weight, preferably 20 to 92% by weight, in particular 30 to 91% by weight, based on the graft polymer, of at least one vinyl monomer on
B.1.2 95 to 5% by weight, preferably 80 to 8% by weight, in particular 70 to 9% by weight, based on the graft polymer, of one or more rubber-elastic graft substrates having glass transition temperatures < -10°C, more preferably < -40°C, particularly preferably < - 70°C, determined by dynamic scanning calorimetry (DSC) according to DIN EN 61006 in the version of 2004 at a heating rate of 10 K/min with determination of Tg as the midpoint temperature (tangent method).

8. The composition of any one of claims 1 to 7, wherein the at least one vinyl monomer B.1.1 is a mixture of
B.1.1.1 65 to 85 % by weight, particularly preferably 70 to 80 % by weight, more preferably 74 to 78 % by weight, in each case based on the sum of B.1.1 and B.1.2, of vinylaromatics and/or ring-substituted vinylaromatics and/or (C₁-C₂₀)-alkyl (meth)acrylates, and
B.1.1.2 15 to 35 % by weight, particularly preferably 20 to 30 % by weight, more preferably 22 to 26 % by weight, in each case based on the sum of B.1.1.1 and B.1.1.2, of vinyl cyanides and/or (C₁-C₂₀)-alkyl (meth)acrylates and/or derivates of unsaturated carboxylic acids.

9. The composition of claims 7 or 8, wherein the rubber-elastic graft substrates B.1.2 of the graft polymers include diene rubbers, EP(D)M rubbers, acrylate, polyurethane, silicone, chloroprene, ethylene/vinyl acetate and also acrylate-silicone composite rubbers.

10. The composition of any one of claims 1 to 9, wherein the rubber-modified graft polymer is acrylonitrile-butadiene-styrene.

11. The composition of any one of claims 1 to 10, wherein the rubber-modified graft polymer B.1 comprises
a first rubber-modified graft polymer B.1-A obtained by emulsion polymerization and
a second rubber-modified graft polymer B.1-B obtained by bulk polymerization.

12. The composition of any one of claims 1 to 11 obtained by a recycling process of a composition comprising a polycarbonate and a rubber-modified graft polymer B.1.

13. The composition of any one of claims 1 to 12 further comprising at least one further polymeric component distinct from B.1 and B.2 and/or a polymer additive.

14. A thermoplastic molding compound obtained from the composition of any one of claims 1 to 13.

15. A molded article obtained from the thermoplastic molding compound of claim 14.
